# EUROPEAN PATENT SPECIFICATION

(11) **EP 1 042 492 B1**
(45) Date of publication and mention of the grant of the patent: **25.11.2009**
(21) Application number: 98965434.8
(22) Date of filing: 22.12.1998
(51) Int. Cl.: C12N 15/85, C07K 16/12, C07K 16/46, C12N 5/10, A61K 39/40

(54) **HUMANIZED MONOCLONAL ANTIBODIES THAT PROTECT AGAINST SHIGA TOXIN INDUCED DISEASE**
HUMANISIERTE MONOKLONALE ANTIKÖRPER DIE SCHÜTZEN GEGEN SHIGATOXININDUZIERTE KRANKHEITEN
ANTICORPS HUMANISES MONOCLONAUX PROTEGEANT CONTRE LES MALADIES INDUITES PAR LES TOXINES DE SHIGA

(30) Priority: 23.12.1997 US 68635 P; 18.12.1998 US 215163
(43) Date of publication of application: 11.10.2000
(73) Proprietor: Sunol Molecular Corporation, Miramar, Florida 33025 (US); HENRY M. JACKSON FOUNDATION FOR THE ADVANCEMENT OF MILITARY MEDICINE, Rockville, MD 20852 (US)
(72) Inventor: Stinson, Jeffrey R., Davie, FL 33331 (US); Wong, Hing, Weston, FL 33332 (US); O'Brien, Alison D., Bethesda, MD 20817 (US); Schmitt, Clare K., Rockville, MD 20853 (US); Melton-Celsa, Angela, Sterling, VA 20165 (US)
(74) Representative: Bird, William Edward
(86) International application number: PCT/US1998/027267
(87) International publication number: WO 1999/032645

(56) References cited:
- EP-A- 0 239 400
- WO-A-98/20903
- H. RÜSSMANN ET AL.: "Variants of Shiga-like toxin II constitute a major toxin component in Escherichia coli O157 strains from patients with haemolytic uraemic syndrome." JOURNAL OF MEDICAL MICROBIOLOGY, vol. 40, no. 5, May 1994, pages 338-343, XP002100300 London, GB
- A. LOBUGLIO ET AL.: "Mouse/human chimeric monoclonal antibody in man: Kinetics and immune response." PROCEEDINGS OF THE NATIONAL ACADEMY OF SCIENCES OF THE U.S.A., vol. 86, June 1989, pages 4220-4224, XP002100301 Washington, DC, USA cited in the application
- J. SPEIRS ET AL.: "Detection of Escherichia coli cytotoxins by enzyme-linked immunosorbent assays." CANADIAN JOURNAL OF MICROBIOLOGY, vol. 37, no. 8, August 1991, pages 650-653, XP002100302 Ottawa, Canada
- PERERA, L.P.: "Isolation and characterization of monoclonal antibodies to Shiga-like toxin II of enterohemorrhagic Escherichia coli and use of the monoclonal antibodies in a colony enzyme-linked immunosorbent assay." JOURNAL OF CLINICAL MICROBIOLOGY, vol. 26, no. 10, October 1988 (1988-10), pages 2127-2121, XP002918490 USA ISSN: 0095-1137
- STROCKBINE N A ET AL.: "Characterization of monoclonal antibodies against Shiga-like toxin from Escherichia coli" INFECTION AND IMMUNITY, vol. 50, no. 3, December 1985 (1985-12), pages 695-700, XP001094992 USA ISSN: 0019-9567
- SCHMITT C K ET AL: "Two copies of Shiga-like toxin II-related genes common in enterohemorrhagic Escherichia coli strains are responsible for the antigenic heterogeneity of the O157:H- strain E32511." INFECTION AND IMMUNITY, vol. 56, no. 3, March 1991 (1991-03), pages 1065-1073, XP002923369 USA
- NAKAO H. ET AL: "Monoclonal antibody to Shiga toxin 2 which blocks receptor binding and neutralizes cytotoxicity." INFECTION AND IMMUNITY, vol. 67, no. 11, November 1999 (1999-11), pages 5717-5722, XP002181517 USA
- BOYD B. ET AL: 'Serological responses to the B subunit of Shiga-like toxin 1 and its peptide fragments indicate that the B subunit is a vaccine candidate to counter action of the toxin.' INFECTION AND IMMUNITY vol. 59, no. 3, March 1991, USA, pages 750 - 757, XP008101487

## Description

### Field of the Invention

Applicants describe a family of multi-unit bacterial proteins that are associated with hemorrhagic colitis and the life-threatening sequela, hemolytic uremic syndrome. These proteins, defined as members of the "Shiga toxin family", have been utilized in the isolation and identification of murine monoclonal antibodies. Applicants further describe the construction of humanized monoclonal antibodies which incorporate the mouse variable regions. Applicants also describe antibodies to Shiga toxins or toxoids, both monoclonal and polyclonal, and their use in treating, diagnosing, and preventing of disease and infections caused by pathogenic *E*. *coli.* The preparation of humanized monoclonal antibodies to proteins of the Shiga toxin family is disclosed.

### Background of the Invention

Enterohemorrhagic *Escherichia coli* (EHEC) are associated with food-borne outbreaks of bloody diarrhea or "hemorrhagic colitis" (HC) and the hemolytic uremic syndrome (HUS). (Spika, J. et al., J. Pediatr., 109: 287-291(1986); Remis, R, Ann. Intern. Med., 101:624-626 (1984); Riley, L. et al., N. Engl. J. Med., 308: 681-685 (1983)). EHEC infection can be deadly and poses a significant threat to the young and the elderly, who are the most likely to develop serious complications from EHEC infections. Several outbreaks and sporadic cases of HC and HUS have occurred over the past few years, with the largest U.S. outbreak in 1993. In that outbreak, over 500 cases of HC and HUS were traced to contaminated hamburgers from a Jack-in-the Box fast food restaurant. (Centers for Disease Control and Prevention, Morbid. Mortal. Weekly Rep., 42:258 (1993)). In July 1996, a large outbreak of EHEC in Japan resulted in over 10,000 infected individuals and 8 deaths. Many Japanese children required hospitalization.

Primarily, HC and HUS are transmitted by the ingestion of contaminated food, particularly undercooked beef products, such as hamburger. (Doyle et al., J. Appl. Environ. Microbiol. 53: 2394 (1987); Samadpour et al., J. Appl. Environ. Microbiol. 60: 1038 (1994)). With the prevalence of EHEC in cattle and the subjective nature of differentiating between cooked and undercooked hamburgers, a stop at a fast food restaurant or a family barbecue can result in tragedy.

HC and HUS appear to be mediated by the toxin produced by EHEC and *Shigella dysenteriae* (for a review, see O'Brien and Holmes, Microbiol. Rev., 51: 206-220 (1987)). These bacteria produce a family of closely related cytotoxins that collectively will be called "Shiga toxins" for the purpose of this application. Shiga toxins (alternatively, "verotoxins") have cytotoxic, and enterotoxic activity (Strockbine, N. et al., Infect. Immun., 53: 135-140 (1986)).

Based on the exhibition of immunological non-cross-reactivity, the Shiga toxins have been divided into two groups. (Strockbine et al., *supra*). These groups have been designated Shiga toxin type 1 (Stx1) and Shiga toxin type 2 (Stx2). The Stx1 group contains the prototype Stx1 toxin from EHEC as well as the Shiga toxin from *Shigella dysenteriae* type 1. In recent years, other types of toxins have been discovered and considered to be members of the Stx2 group. These are Stx2e, Stx2c, and Stx2d. (Lindgren et al., Infection and Immunology, 61: 3832 (1993); Schmitt, C. et al., Infect. Immun., 59: 1065-1073 (1991); Marques, L. et al., FEMS Lett., 44: 33-38 (1987)).

For the purposes of this application the term "Shiga toxin" encompasses Shiga toxin and any other toxins in the Stx1 or Stx2 group or their variants. The abbreviation "Stx" will refer to the toxin protein itself.

Despite this knowledge about the results of exposure to these toxins, currently there is no known cure or vaccine for HC or HUS. Antibiotics may even make the severe complications worse by increasing toxin release from bacteria. Thus, there is a need for a compound to prevent or to treat the complications of EHEC produced by Shiga toxin. Such a compound could be used to treat infected patients and decrease the systemic effects of toxin on the CNS, blood and kidneys. In addition, if the toxin could be neutralized, antibiotics could be safely given to kill the bacteria in the GI tract. Such a compound could also be used to prevent infectious complications, by treating exposed or high risk individuals before they acquire EHEC infection. Such individuals would include children in day care or the elderly in nursing homes, where a case of EHEC diarrhea has been detected. These individuals are at increased risk to develop severe complications and spread of EHEC in these environments is not unusual.

The knowledge of the immunological cross reactivity of the Shiga toxins offers a tantalizing prospect for pharmacological approaches to EHEC treatment. Currently, there are no known prophylactic or therapeutic agents available for this disease. Accordingly, there is a need in the art to provide monoclonal antibodies that can bind to Shiga toxins which could prevent or lessen the devastating effects of these toxins. There is also a need in the art for data concerning the binding site of such antibodies so that other antibodies with similar abilities can be identified and isolated.

Monoclonal antibodies against Shiga-like toxins have been described in Strockbine et al., Infection and Immunity, 1985, 50: 695-700 and in Perera, et al J. Clinical Microbiol., 1988,26: 2127-2131. Strockbine et al disclose that the use of the13C4 antibody neutralises the lethality for mice induced with Shiga-like toxin from *E.coli* H30. Strockbine et al further show the use of the 13C4 for the detection of bacterial colonies producing high levels of Shiga-like toxins. Perera et al. describes the production of five mouse monoclonal antibodies (including the antibody 11E10) that were capable of neutralising the cytotoxicity of Shiga-like toxin II. WO9820903 , published on May 22, 1998 describes therapeutic methods for the treatment of hemolytic uremic syndrome, by administering of monoclonal antibodies, chimeric monoclonal antibodies and monospecific polyclonal antibodies specific for Shiga-like toxin.

There is a related need in the art for humanized or other chimeric human/mouse monoclonal antibodies. In well publicized studies, patients administered murine anti-TNF (tumor necrosis factor) monoclonal antibodies developed anti-murine antibody responses to the administered antibody. (See, e. g., Exley A. R., et al., Lancet 335: 1275-1277 (1990)). This type of immune response to the treatment regimen, commonly referred to as the HAMA response (for human anti-mouse antibodies), decreases the effectiveness of the treatment and may even render the treatment completely ineffective. Humanized or chimeric human/mouse monoclonal antibodies have been shown to significantly decrease the HAMA response and to increase the therapeutic effectiveness. (LoBuglio et art., P.N.A.S. 86: 4220-4224 (June 1989)). Methods to humanize antibodies comprise the exchange of constant regions (see for example Lobuglio et al, supra) or CDR grafting (EP0239400)

### Summary of the Invention

The present invention relates to a pharmaceutical composition comprising a first humanized monoclonal antibody that binds to Shiga toxin type 1 (Stx1) protein and a second humanized monoclonal antibody that binds to Shiga toxin type 2 (Stx2) protein, wherein the first antibody comprises a human immunoglobulin constant region and comprises the immunoglobulin heavy chain and light chain variable regions as set forth in SEQ ID NOs: 19 and 21 and wherein the second antibody comprises a human immunoglobulin constant region and comprises the immunoglobulin heavy chain and light chain variable regions as set forth in SEQ ID NOs: 42 and 44.

The present invention further relates to pharmaceutical composition comprising a first humanized monoclonal antibody that binds to Stx1 and a second humanized monoclonal antibody that binds to Stx2 antigen, wherein the variable region of the second antibody comprises the variable region of the murine 11E10 antibody with ATCC Accession No. CRL 1907 and the variable region of the first antibody comprises the variable region of the murine 13C4 antibody with ATCC Accession No. CKL 1794.

In particular embodiments of the above-described pharmaceutical compositions, the first or second antibody comprises a human constant region selected from the group consisting of IgG, IgA, and IgM.

In other particular embodiments of the above-described pharmaceutical compositions the human constant region of the first or second monoclonal antibody is IgG.

In yet other particular embodiments of the above-described pharmaceutical compositions the human constant region of the first or second monoclonal antibody is IgG1-kappa.

Optionally, the above-described pharmaceutical composition, further comprises a pharmaceutically acceptable carrier or diluent.

The present invention further relates to the use of a composition comprising a first humanized monoclonal antibody that binds to Stx1 protein and a second humanized monoclonal antibody that binds to Stx2 protein, wherein the first antibody comprises a human immunoglobulin constant region and comprises the immunoglobulin heavy chain and light chain variable regions as set forth in SEQ ID NOs: 19 and 21 and wherein the second antibody comprises a human immunoglobulin constant region and comprises the immunoglobulin heavy chain and light chain variable regions as set forth in SEQ ID NOs: 42 and 42 in the manufacture of a medicament for the treatment or prevention of an infection resulting in hemolytic uremia syndrome caused by Enterohemorrhagic *Escherichia coli* or other Shiga toxin producing bacteria.

The present invention also relates to the use of a composition comprising a first humanized monoclonal antibody that binds to Stx1 and a second humanized monoclonal antibody that binds to Stx2 antigen, wherein the variable region of the second antibody comprises the variable region of the murine 11E10 antibody with ATCC Accession No. CRL 1907and the variable region of the first antibody comprises the variable region of the murine 13C4 antibody with ATCC Accession No. CKL 1794 in the manufacture of a medicament for the treatment or prevention of an infection resulting in hemolytic uremia syndrome caused by enterohemorrhagic *Escherichia colior* other Shiga toxin producing bacteria.

In particular embodiments the first or second antibody comprises a human constant region selected from the group consisting of IgG, IgA, and IgM.

In other particular embodiments the human constant region of the first or second monoclonal antibody is IgG, preferably IgG1-kappa.

To satisfy these needs in the art, applicants provide humanized mouse monoclonal antibodies to Shiga toxin 1 and Shiga toxin 2, and in addition sets forth the variable region associated with Shiga toxin neutralization as well as the specific complementarity determining regions (CDRs) of the variable region Human monoclonal antibodies are provided, derived from the mouse monoclonal antibodies 13C4 and 11E10, which are specifically reactive with Stx1 and Stx2, respectively.

The accompanying drawings are included to provide a further understanding of the invention.They illustrate the invention and, together with the description, serve to explain the principles of the invention.

### Brief Description of the Drawings

Some of the following embodiments have been included for reference purposes only and do not fall under the scope of the claims.
Figure 1 provides a schematic representation of the general cloning strategy used for cloning the variable region gene fragments of both the 11E10 and 13C4 antibodies.
Figure 2 lists the oligonucleotide sequences used for PCR amplification of the variable regions of the 13C4 antibody.
Figure 3 recites the DNA and amino acid consensus sequences for the variable regions of both the heavy and light chains of the 13C4 antibody. The DNA consensus sequence was determined using automate fluorescent dye-terminator sequence reactions of the amplified cDNAs analyzed with an ABI 373 sequencing instrument. The CDRs were identified according to the nomenclature of Kabat and Wu (Kabat, et al. (1991), Sequences of Proteins of Immunological Interest, Vol. I, 5th edition, U.S. Department of Health and Human Services) and are located as follows:

| | |
|---|---|
| Heavy Chain CDRs: (SEQ ID NO: 19) | CDR1-aa31-35 |
| | CDR2-aa50-66 |
| | CDR3-aa99-111 |
| | |
| Light Chain CDRs: (SEQ ID NO: 21) | CDR1-aa24-34 |
| | CDR2-aa50-56 |
| | CDR3-aa89-97 |

Figure 4 illustrates the strategy for construction of the mammalian expression vector tKMC249A containing the variable regions of the 13C4 antibody.
Figure 5 lists the oligonucleotides used for PCR amplification of the variable regions of the 11E10 antibody.
Figure 6 recites the DNA and amino acid consensus sequences for the variable regions of both the heavy and light chains of the 11E10 antibody. The DNA consensus sequence was determined using automate fluorescent dye-terminator sequence reactions of the amplified cDNAs analyzed with an ABI 373 sequencing instrument. The CDRs were identified according to the nomenclature of Kabat and Wu (Kabat and Wu; *supra*) and are located as follows:

| | |
|---|---|
| Heavy Chain CDRs: (SEQ ID NO: 44) | CDR1-aa31-35 |
| | CDR2-aa50-66 |
| | CDR3-aa99-108 |
| | |
| Light Chain CDRs: (SEQ ID NO: 42) | CDR 1-aa24-40 |
| | CDR2-aa56-62 |
| | CDR3-aa95-103 |

Figure 7 illustrates the strategy for construction of the mammalian expression vector pACE4 containing the variable regions of the 11E10 antibody.
Figure 8 shows the anti-Stx1 13C4 antibody production ELISA and demonstrates that the H13C4 cell line produces an antibody consisting of human IgG and kappa constant domains.
Figure 9 shows the anti-Stx1 13C4 toxin binding activity ELISA and demonstrates that the antibody produced by the H13C4 cell line binds to Stx1 toxin.
Figure 10 shows the anti-Stx2 11E10 antibody production ELISA and demonstrates that the H11E10 cell line produces an antibody consisting of human IgG and kappa constant domains.
Figure 11 shows the anti-Stx2 11E10 toxin binding activity ELISA and demonstrates that the antibody produced by the H11E10 cell line binds to Stx2 toxin.
Figure 12 illustrates the strategy for construction of the plasmid pCDNA3mut.LCPL.LCVK.
Figure 13 illustrates the strategy for construction of the plasmid pCDNA3mut.HCPL.HCV2b.
Figure 14 illustrates the strategy for construction of the plasmids pSUN9 and pSUN9:kappa.
Figure 15 illustrates the strategy for construction of the plasmids PSUN10 and pSUN10:IgG1.
Figure 16 illustrates the strategy for construction of the plasmid pJRS311.
Figure 17 illustrates the strategy for construction of the plasmid pJRS315.

### Detailed Description of the Invention

Applicants disclose humanized monoclonal antibodies that bind to Shiga toxin proteins, and the use of such antibodies in the treatment or prevention of Shiga toxin- induced diseases. Antibodies are proteinaceous structures made up of two heavy and two light chains. The five different classes of higher vertebrate antibodies-IgM, IgD, IgG, IgA and IgE-are distinguished by their heavy chains (mu, delta, gamma, alpha and epsilon, respectively). Each class has further subclasses; IgG, for example, may be IgG1, IgG2, IgG3, or IgG4, where the heavy chain is gamma 1, gamma 2, gamma 3 or gamma 4, respectively. The pair of light chains in these classes or subclasses may be either kappa or lambda.

Antibodies are further divided into a constant region and a variable region. For both the heavy and light chains, the carboxy-terminal ends make up the constant sequence region, while the amino terminal ends contain the variable sequence region. Within these variable regions, the complementarity determining regions (CDRs) are located which are primarily responsible for the observe antigen binding which is characteristic of antibodies.

"Humanized" monoclonal antibodies mean monoclonal antibodies originally from a non-human source to which human components have been substituted. In particular, humanized monoclonal antibodies comprise variable regions which derive from non-human sources and constant regions which derive from human sources.

As set forth above, Shiga toxin proteins (Stx) refer to the family of multi-unit bacterial proteins produced by EHEC and *Shigella dysenteriae* which are associated with outbreaks of Shiga toxin induced diseases. Shiga toxin proteins are meant to encompass Stx of *Shigella dysenteriae*-type 1 and Stx type 1 and type 2, and type 2 variant toxins of *E*. *coli.*

Shiga toxin-induced diseases of humans include bloody diarrhea, hemorrhagic colitis, hemolytic uremic syndrome, and thrombotic thrombocytopenic purpura.

Applicants further disclose humanized monoclonal antibodies that have the same binding specificity of at least two well characterized murine monoclonal antibodies. These two monoclonal antibodies were developed in Dr. Alison O'Brien's laboratory as set forth in Strockbine, N. A. et al., Infection and Immunity, 1985, 50: 695-700 and Perera, L. P. et al., J. Clinical Microbiol., 1988,26: 2127-2131, and have been deposited at the ATCC as set forth below.

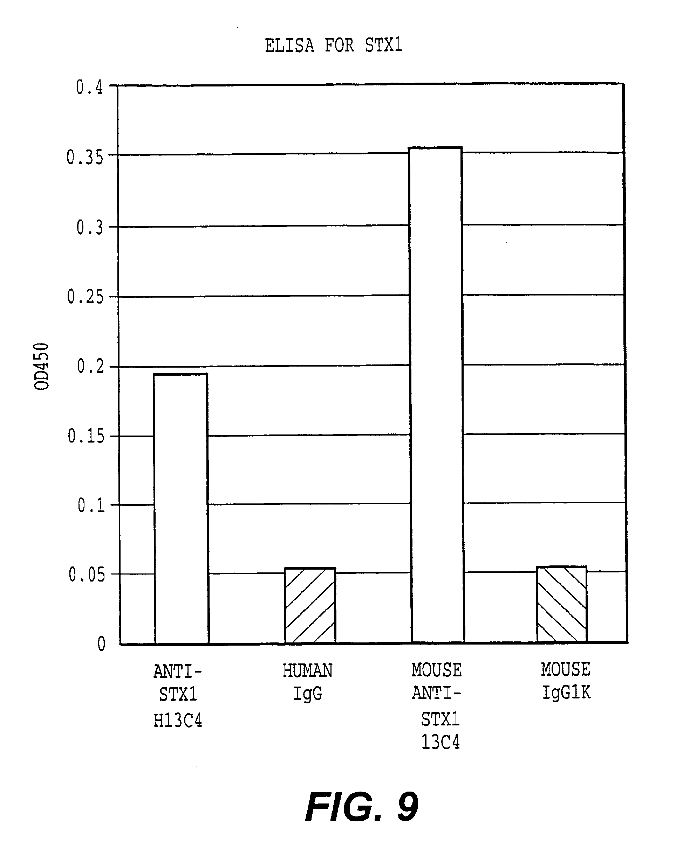

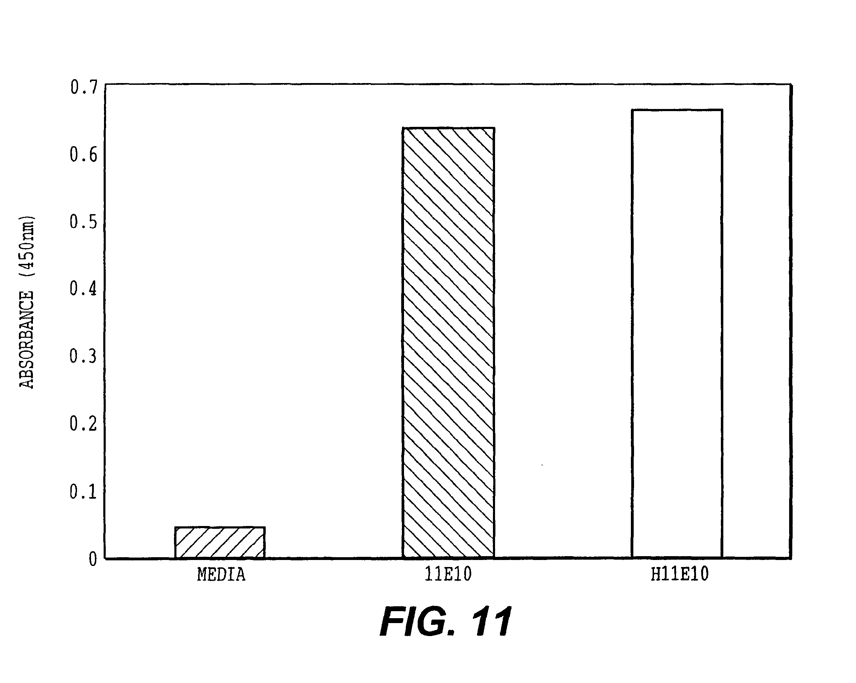

By "same binding specificity" is meant a level of binding sufficiently detectable in a standard binding assay to distinguish between toxin binding and non-specific background binding as exemplified by appropriate controls (for examples, see Figure 9 and Figure 11). Those of ordinary skill in the art can readily test for binding levels using routine skills and techniques.

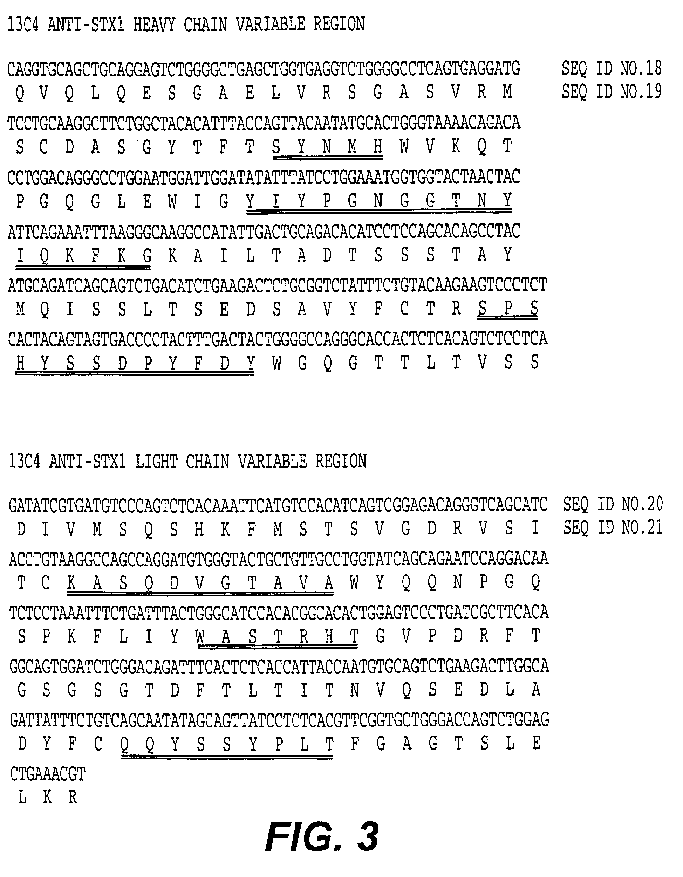

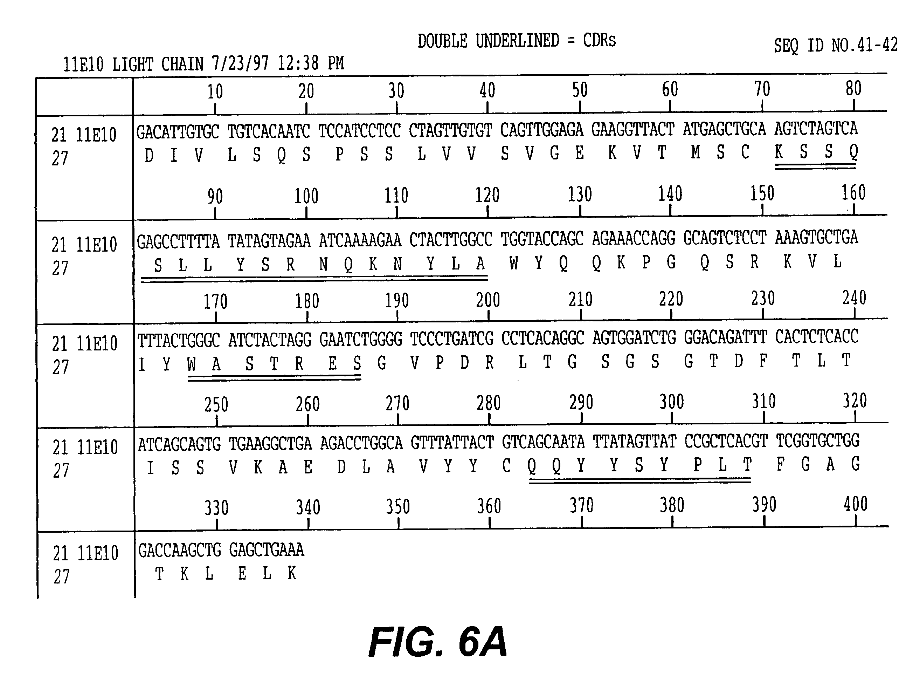

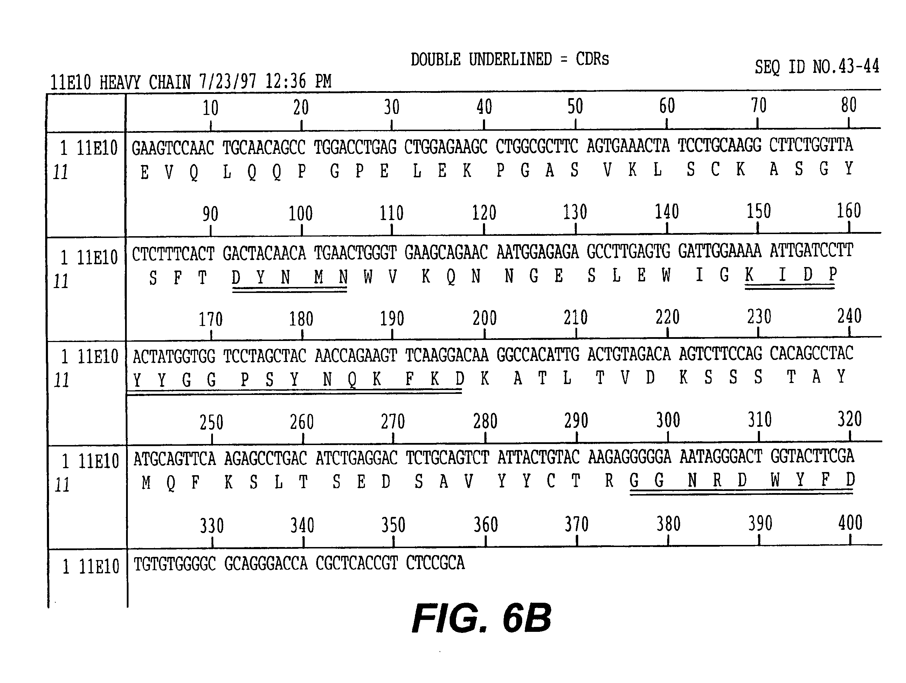

In another preferred embodiment, the humanized antibodies are characterized by their structural features. In one aspect of this embodiment, the constant region is a human constant region and the variable region derives from a rodent, preferably a mouse. Although not limited to these, the four variable regions set forth in the specification, particularly in Figure 3 and Figure 6, are preferred. Of course, such variable regions may include modifications (i. e., deletions, additions, and substitutions) that do not appreciably diminish the characteristic binding associated with the exemplified variable regions.

In yet another embodiment, the CDRs of the variable region are employed.. As noted above, CDRs are located in the variable regions of both the light and heavy chains and are responsible for antigen binding. In view of the ready ability of those in this art to determine the CDR regions, the present disclosure is not limited to the CDRs specifically set forth. Indeed, the vectors created by the applicants and described in great detail below are sufficient for use with any CDRs of non-human antibodies to Shiga toxins.
For example the humanized monoclonal antibody derives its constant regions from IgG1-kappa and its variable regions from all or part of the sequences as set forth in Figures 3 and 6. The expression vectors which code for these monoclonal humanized antibodies, and the host cells which have been transformed with such expression vectors are described in the Examples. Finally, the invention comprises pharmaceutical compositions as claimed and described in the "summary of the invention". These pharmaceutical compositions include but are not limited to diluents and carriers known in the art, such as saline and sucrose solutions appropriate for application to patients. As used herein, "patients" refers to any susceptible mammal, such as dogs, horses, mice, etc., but is particularly preferred to mean humans.

Methods of treatment involve the administration of a therapeutically effective mount, as well as a prophylatically effective amount, of the humanized monoclonal antibody. As those in the art would recognize, a therapeutically effective amount is a dose that ameliorates edema, thrombocytopenia, and uremia associated with EHEC-mediated HUS. Similarly, a prophylactically effective amount is a dose that prevents exposed individuals from developing these symptoms.

The following examples illustrate specific embodiments of the invention. Some of these have been included for reference purposes only and do not fall under the scope of the claims.

### Humanization of the Anti-Stx1 antibody 13C4

The following examples 1-3 relate to anti-Stx1 antibody 13C4 and its humanized counterpart H13C4.

### EXAMPLE 1. Cloning of the 13C4 variable region cDNAs.

The hybridoma cell producing the"13C4"antibody (Anti-Stx1) was deposited on December 2,1987, at the American Type Culture Collection, Rockville, MD under Accession No. CRL 1794, and can be obtained from the ATCC, or, as here, from Dr. Alison O'Brien (for details of hybridoma preparation, see Strockbine, N. A. et al., Infection and Immunity, 50: 695-700 (1985)). A vial of cells was thawed and resuspended in IMDM (Mediatech) complete media supplemented with 10% FBS (Irvine).

Total RNA was isolated from 1x10⁷ "13C4" cells using the Midi RNA Isolation kit (Qiagen) following the manufacturer's procedure. The RNA was dissolve in 10 mM Tris, 0.1 mM EDTA (pH 8.4) containing 0.03 U/µg Prime RNase Inhibitor (5'-3') to a final concentration of 0.25 µg/µl.

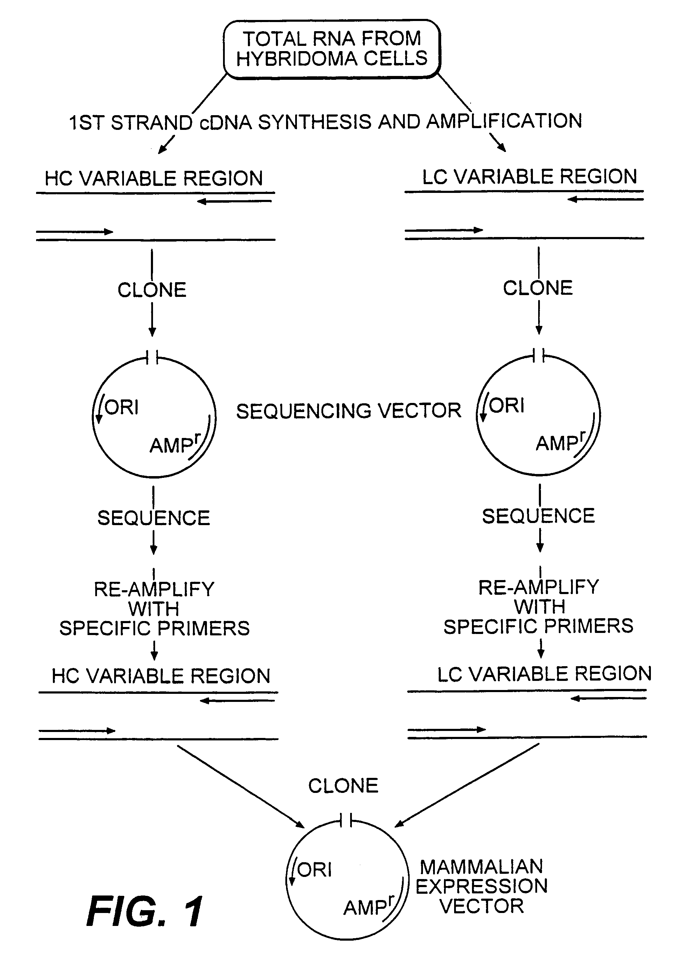

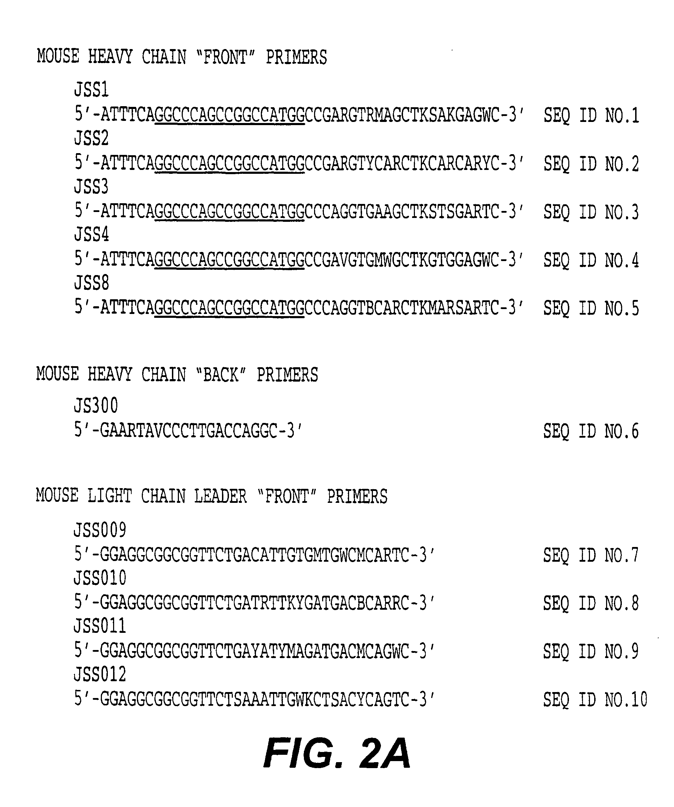

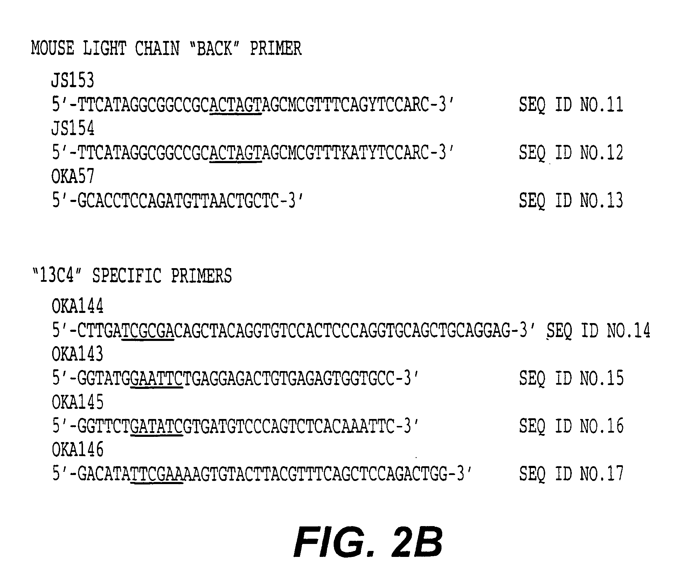

Figure 1 shows the strategy for cloning the variable region gene fragments and Figure 2 lists the oligonucleotide primers used. The "13C4" total RNA (2 µg) was converted to cDNA by using Superscript 11-MMLV Reverse Transcriptase (Life Technologies) and mouse kappa (oKA57, SEQ ID NO: 57) and mouse CH1 (JS300, SEQ ID NO: 6) specific priming, according to the manufacturer's procedures. The first strand cDNA synthesis products were then purified using a membrane concentrator device (Amicon Centricon 30 or Millipore UltraFree 15). Of the cDNA recovered, 3 µl was used as template DNA for PCR. Typical PCR amplification reactions (100 µl) contained template DNA, 50 pmoles of the appropriate primers (JSS9, JSS10,JSS11,JSS12,JS153 & JSS154-SEQ ID NO: 9-12 for light chains; JSS1, JSS2, JSS3, JSS4, JSS8 and aKA-143SEQ ID NO: 1-5 and SEQ ID NO: 15 for heavy chains), 2.5 units of ExTaq polymerase (PanVera), 1x ExTaq rection buffer, 200 µM dNTP, and 2mM MgCl₂. Each of the templates was denatured by an initial incubation at 96 °C for 1 min. The heavy chain products were amplified by 40 or 50 thermal cycles of 59 to 72°C for 30 sec., 72°C for 30 sec., then 96°C for I min. and a final extension step at 72°C for 5 min. The light chain products were amplified by 6 thermal cycles of 46,48 or 54°C for 30 sec., 72°C for 30 sec., then 96°C for 1 min followed by 35 step cycles of 60°C for 1 min, then 96°C for 1 min. and a final extension step at 72°C for 5 min. The PCR products from the successful reactions were purified using the Wizard PCR Purification system (Promega) as per the manufacturer's procedure.

The heavy chain and light chain PCR products (approximately 400 bp) were then cloned into a bacterial vector for DNA sequence determination. Ligations of the PCR fragments were carried out into the pCR2.1 T/A style cloning vector (Invitrogen) following the manufacturer's procedures using 1: 1, 3: 1 and 5: 1 insert to vector molar ratios. One half of each of the ligation reactions was used to transform competent XL1 Blue cells.

Heavy chain plasmid clones containing DNA inserts were identified using diagnostic restriction enzyme digests with EcoRI (New England Biolabs). The DNA sequence of plasmids (tKMC217B) containing inserts of the appropriate size (400 bp) were then determined. The final consensus DNA sequence of the heavy chain variable regions is shown in Figure 3.

The light chain PCR products were identified differently. The hybridoma cell line that expresses the "13C4" antibody was made by fusing mouse splenocytes with the SP2/0 myeloma cell line. The SP2/0 cell line transcribes a pseudogene for the kappa light chain. The pseudogene transcript, when converted to cDNA by RT-PCR, contains an AflIII restriction site. Light chain candidate clones (tKMC226 & 227) were digested with AflIII (New England Biolabs) using the manufacturer's procedures to identify clones containing inserts of the appropriate size (403 bp; no AflIII site is present in these products). The final consensus DNA sequence of the light chain variable regions is shown in Figure 3 with the CDRs indicated by underlining.

### EXAMPLE 2. Construction of the expression vector tKMC249A.

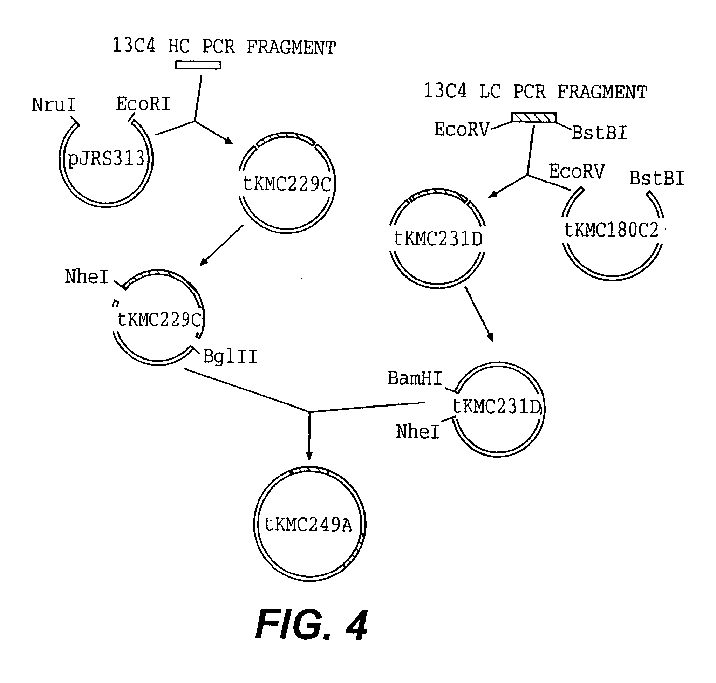

The heavy and light chain variable regions were then subcloned into mammalian expression plasmid vectors for the production of recombinant chimeric mouse/human antibody molecules. The vectors result in the production of recombinant antibody molecules under the control of CMV transcriptional promoters. The heavy chain molecules are direct cDNA constructs that fuse the variable region sequence directly into the human IgG 1 constant domain. The light chain molecules, on the other hand, have a mouse kappa intron region 3' of the variable region coding fragment. After splicing, the variable region becomes fused to a human kappa constant region exon (Figure 4). The selectable marker for the vector in mammalian cells is aminoglycoside phosphotransferase (neo), using the drug G418 (CellTech).

### A. Creation of the expression vectors

To create the heavy and light chain expression vectors required DNA fragment ligations and site directed mutagenesis steps. The result was vectors that express both antibody chains with CMV promoter driven transcription. Neomycin resistance serves as a dominant selectable marker for transfection of mammalian cells. In addition, these vectors have been designed to allow convenient cloning of any light chain variable region as EcoRV/BstBI fragment, any heavy chain variable region as a NruI/EcoRI fragment, and any heavy chain constant domain as an EcoRI/NotI fragment. These restriction sites were chosen because they occur rarely (if ever) in human and mouse variable regions. There is a mouse J region/kappa intron fragment fused to a human kappa exon so that after post-transcriptional splicing a mouse human chimeric kappa light chain is produced. Lastly, the vectors were designed to facilitate excision (BglII/NheI) of a whole antibody expression cassette from one vector to be ligated into a second vector cut with BamHI/NheI, creating an expression vector with the apparatus for both chains.

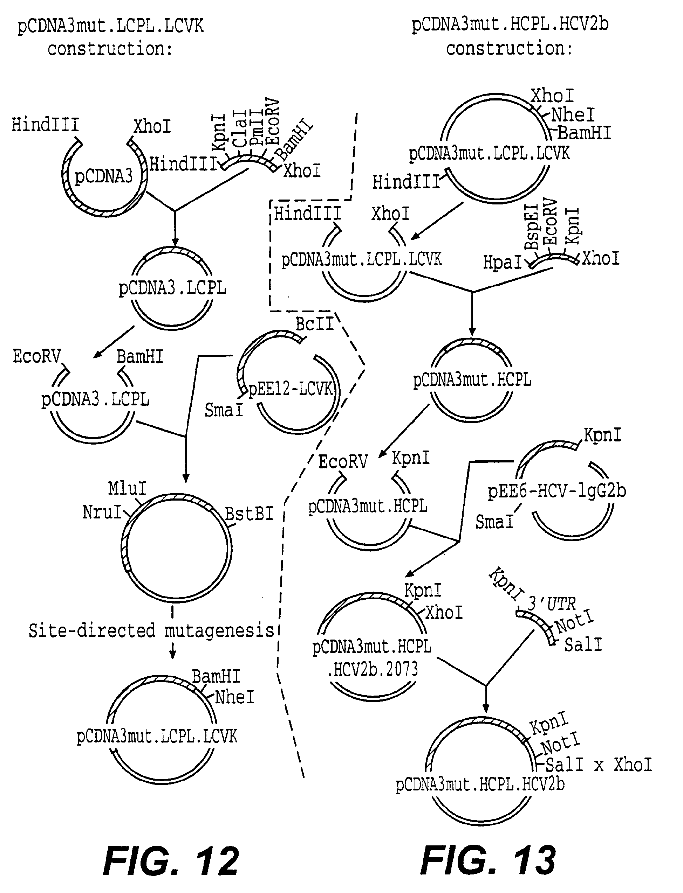

The backbone of the vector was the plasmid pCDNA3 (Invitrogen). This plasmid was cut with HindIII/XhoI and a "light chain polylinker" DNA fragment was inserted to create the starting "light chain vector" pCDNA3.LCPL (see Figure 12). This linker contained the restriction sites HindIII, KpnI, ClaI, PmlI, EcoRV, XmaI, BamHI and XhoI to facilitate subsequent cloning steps. A SmaII/BclI DNA fragment containing a light chain leader, mouse anti-CKMB kappa light chain genomic fragment, and 3'UTR was cloned into the EcoRV/BamHI sites of pCDNA3.LCPL. The mouse kappa intron, exon, and 3'UTR in this fragment was derived from LCPXK2 received from Dr. Richard Near (Near, R. I. et al., 1990, Mol. Immunol. 27: 901-909). Mutagenesis was then performed to eliminate an NruI (209), MluI (229), and BstBI (2962) and to introduce an NheI (1229) and a BamHI (1214) site to create the plasmid pCDNA3mut. LCPL. LCVK (see Figure 12).

A second "heavy chain vector" pCDNA3mut.HCPL was constructed from the pCDNA3mut.LCPL.LCVK plasmid by replacing the light chain expression region (HindIII/XhoI) with a "heavy chain polylinker" consisting of restriction sites HpaI, BspEI, EcoRV, KpnI, and XhoI. This plasmid was digested with EcorRV and KpnI. A SmaI/KpnI digested DNA fragment containing a heavy chain leader and an anti-CKMB IgG2b mouse heavy chain genomic fragment was then ligated into the EcoRV/KpnI digested plasmid. A KpnI/SalI oligonucleotide fragment containing a 3'UTR and a NotI upstream of the SalI site was subsequently cloned into the KpnI/XhoI digested plasmid, (knocking out the XhoI site), to create the plasmid pCDNA3mut.HCPL.HCV2b (see Figure 13).

A human kappa light chain constant domain was then cloned into pCDNA3mut.LCPL.LCVK as a EcoNI/XhoI fragment generating the plasmid tKMC 180C2. A human IgG1 constant domain was cloned into PSLTN 10 as a EcoRI/NotI fragment creating the plasmid pJRS313. The variable regions of 13C4 were cloned into these two vectors (as described above). A BglII/NheI fragment from the human heavy chain vector tKMC229C was then cloned into the human light chain vector tKMC231D cut BamHI/NheI to create tKMC249A (see Figure 4).

### B. Subcloning the variable regions into the expression vectors

The variable region gene fragments were re-amplified by PCR using primers that adapted the fragments for cloning into the expression vectors (see Figures 2 and 4). The heavy chain front primer (oKA143, SEQ ID NO: 15) includes a 5' tail that encodes the C-terminus of the heavy chain leader and an NruI restriction site for cloning, while the heavy chain reverse primer (oKA144, SEQ ID NO: 14) adds a 3' EcoRI restriction site for cloning. The light chain front primer (oKA145, SEQ ID NO: 16) introduces an EcoRV restriction site at the N-terminus of the light chain variable region for cloning, while the light chain reverse primer (oKA146, SEQ ID NO: 17) adds a 3' DNA sequence for the joining region-kappa exon splice junction followed by a BstBI restriction site for cloning.

PCR reactions were performed with reagents as described above and with templates of 1-2 ng of PvuI (New England Biolabs) digested plasmid; each of these templates was denatured by an initial one minute incubation at 96°C. The heavy chain products were amplified by 35 thermal cycles of 55 or 60°C for 30 sec., 72°C for 30 sec., then 96°C for 1 min and a final extension step at 72°C for 5 min. The light chain products were amplified by 8 thermal cycles of 55 or 60°C for 30 sec., 72°C for 30 sec., then 96°C for 1 min followed by 30 step cycles of 60°C for 1 min, then 96°C for 1 min, and a final extension step at 72°C for 5 min.

The 13C4 heavy chain PCR product (approximately 400 bp) was purified using Qiaquick PCR Purification columns (Qiagen) as described by the manufacturer's instructions and subsequently digested with NruI and EcoRI (New England Biolabs). The digested PCR products were purified using the Wizard PCR Purification system (Promega) as per manufacturer's procedure and ligated into NruI/EcoRI digested and gel-purified pJRS313, resulting in plasmid tKMC229C (see Figure 4). The final consensus DNA sequence of the heavy chain variable region and proper splicing of the restriction sites were confirmed in this construct.

The 13C4 light chain PCR product (approximately 350 bp) was purified using Qiaquick PCR Purification columns (Qiagen) as described by the manufacturer's instructions and subsequently digested with EcoRV and BstBI (New England Biolabs). The digested PCR products were purified using Qiaquick PCR Purification columns (Qiagen) as per manufacturer's procedure and ligated into EcoRV/BstBI digested and gel-purified tKMC 180C2 (as described above), resulting in plasmid tKMC231D (see Figure 4). The final consensus DNA sequence of the light chain variable region and proper splicing of the restriction sites were confirmed in this construct.

### EXAMPLE 3. Stable production of recombinant chimeric mouse/human 13C4 antibody.

### A. Transfection of NSO Cells

The plasmid tKMC249A was transfected into NSO cells (Baxter International, Inc., Durant, CA) using electroporation after linearization with PvuI (New England Biolabs). 40 micrograms of the digested plasmid was mixed with 1x107 cells in a total volume of 800 microliters in a 0.4 centimeter cuvette and subjected to a pulse of 250mA, 960µF. The cells were plated out after 24 hours into 96-well tissue culture plates, 6 plates with 200 µl/well, and incubated at 37°C and 10% CO₂. As colonies appeared, the supernatants were assayed for the production of "humanized" antibody and for the capability of the expressed antibody to bind to Stx1.

### B. Assay for Antibody Production

Antibody production and activity assays for the stable transfectants were performed as described below. These assays demonstrate that the transfection of cells with this plasmid construct can result in the production of a stable cell line that produces a humanized chimeric version of the 13C4 mouse hybridoma antibody (designated H13C4).

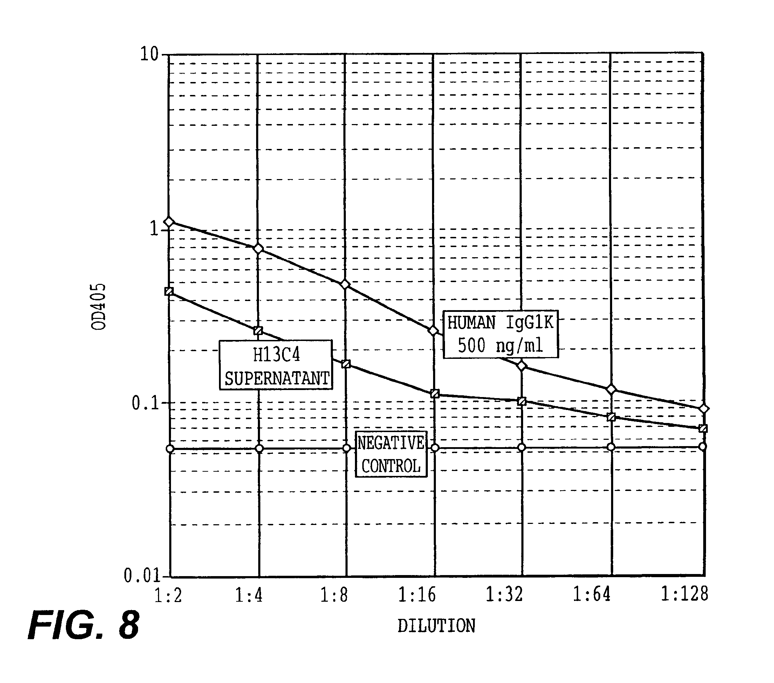

Antibody production ELISA assays were performed in 8-well strips from 96-well microtiter plates (Maxisorp F8; Nunc, Inc.) coated at a 1:500 dilution with Goat anti-Human IgG antibody (Pierce or Biodesign International) using a Tris-HCl coating buffer, pH 8.5. The plates were covered and incubated overnight at 4°C. Plates were then washed once with a rash storage buffer (Tris-HCl/NaCl/0.1% NaN₃). 50 microliters of culture supernatant was then applied to each well that had been filled previously with 50 microliters of a sample/conjugate diluent (Tris-HCl/NaCl/gelatin/Tween-20). The plates were allowed to incubate for 30 to 60 minutes on a rotator at room temperature. They were then washed three times with a wash solution (imidazole/NaCl/Tween-20). A goat anti-human kappa-HRP (Southern Biotechnologies) conjugate was diluted 1: 250 in the sample/conjugate diluent and 100 microliters was added to the wells. The plates were incubated on a rotator for 30 to 60 minutes at room temperature. They were washed 6 times using the wash buffer, and then incubated with 100 µL/well of ABTS developing substrate (Kirkgaard & Perry Laboratories) for 8 minutes at room temperature. The rection was stopped with 100 µL/well of diluted Quench buffer (Kirkgaard & Perry Laboratories). The absorbance value at 405 nm was determined using an automate microtiter plate ELISA reader (Ceres UV9OOHI Bioteck, Winooski, Vermont). The controls for the ELISA assay were a human IgG1K myeloma protein (Biodesign International) and supernatant collected from non-transfected NSO cells. This assay (see Figure 8) demonstrates that the transfection of cells with this plasmid construct results in the cells producing a molecule containing both human heavy chain (IgG) and light chain (kappa) domains.

The supernatants were then assayed for the ability of the expressed antibodies to bind to Stx1 protein by ELISA. The activity assays were preformed in 8-well strips from 96-well microtiter plates (Maxisorp F8; Nunc, Inc.) coated with approximately 0.1 µg/well purified Stx1 received from Alison O'Brien (or obtained as described in Example 7). The plate coating and ELISA procedure was performed in the same manner as the antibody assay above with the substitution of TMB (Kirkgaard & Perry Laboratories) for ABTS as a developing substrate. The absorbance value at 450 nm was determined using an automate microtiter plate ELISA reader (Ceres UV900HI, Bioteck, Winooski, Vermont). As a positive control, the original mouse monoclonal antibody 13C4 was used, and assayed with a goat anti-mouse IgG conjugate (Jackson Laboratories) at a 1: 2000 dilution. This assay (see Figure 9) demonstrates that the transfection of cells with this plasmid construct results in cells producing immunoglobulin that binds to Stx1. Neither mouse nor human IgG I K lacking the anti-Stx variable region bound the toxin.

### Humanization of the EHEC Anti-Stx2 antibody: 11E10

The following examples 4-7 relate to anti-Stx2 and-Stx2 variant antibody 11E10 and its humanized counterpart H11E10. EXAMPLE 4. Cloning of the 11E10 variable region cDNAs.

The hybridoma cell line producing the "11E10" antibody (Anti-Stx2) was deposited on August 1, 1990, at the American Type Culture Collection, Rockville, MD under Accession No. CRL 1907, and can be obtained from the ATCC, or, as here, from Dr. Alison O'Brien (for details of hybridoma preparation, see Perera, L. P. et al., J. Clinical Microbiol., 26: 2127-2131 (1988)). A vial of the cell line was thawed, washed with serum free medium and then resuspended in IMDM (Mediatech) complete media supplemented with 10% FBS (Irvine).

Total RNA was isolated from 1x107 "11E10"cells using the RNeasy RNA Isolation kit (Qiagen) following the manufacturer's procedure. The RNA was dissolve in 10 mM Tris, 0.1 mM EDTA (pH 8.4) containing 0.03 U/µg Prime RNase Inhibitor (5'-3') to a final concentration of 0.63 µg/µl.

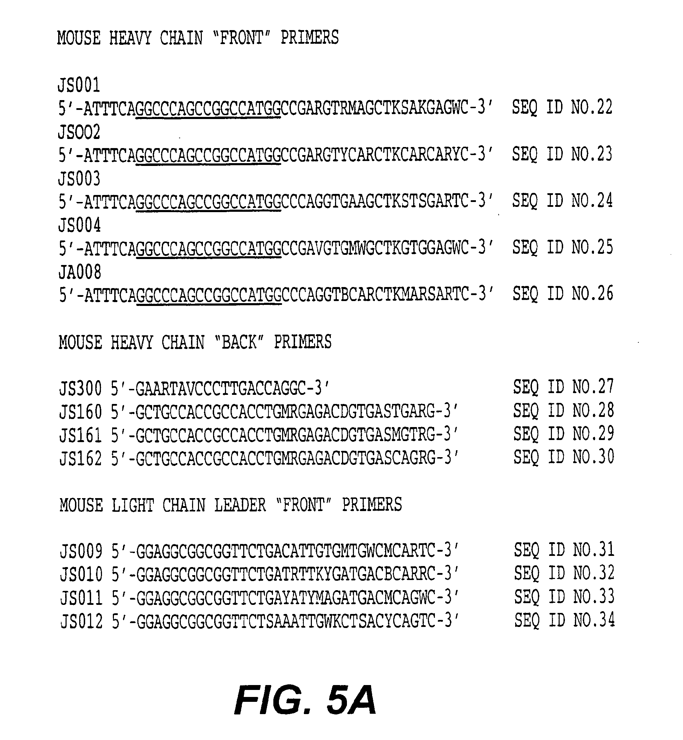

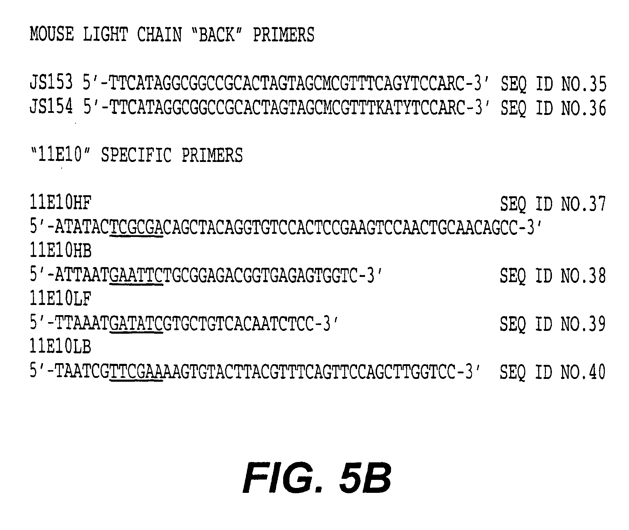

Figure 1 shows the strategy for cloning the variable region gene fragments and Figure 5 lists the oligonucleotide primers used. The "11E10" total RNA (2.5 µg) was converted to cDNA by using Superscript 11-MMLV Reverse Transcriptase (Life Technologies) according to manufacturer's procedures. The mouse light chain (JS153, JS154, SEQ ID NO: 11 and 12) and mouse heavy chain (JS300, SEQ ID NO: 6) were used as specific primers. The first strand cDNA synthesis products were then purified using a Centricon-30 concentrator device (Amicon). Of the 70 µl of cDNA recovered, 3.5 µl was used as template DNA for PCR. Typical PCR amplification reactions (100 µl) contained template DNA, 50 pmoles of the appropriate primers (JS153, JS154 and JS009, JS010, JS011, JS012, SEQ ID NO: 7-12 for light chains, JS160, JS161, JS162 and JS001, JS002, JS003, JS004, JS008, SEQ ID NO: 28-30, SEQ ID NO: 2-5 for heavy chains), 2.5 units of ExTaq polymerase (PanVera), 1x ExTaq reaction buffer, 200 µM dNTP, and 2 mM MgCl₂. The template was denatured by an initial five minute incubation at 96°C. The products were amplified by 35 thermal cycles of 96°C for 1 min., 55°C for 30 sec., and 72°C for 30 sec, followed by 5 min. at 72°C. The PCR products from the successful reactions were purified using the Wizard PCR Purification system (Promega) as per manufacturer's procedure.

The heavy chain PCR products (approximately 400 bp) and the light chain PCR products (approximately 350 bp) were then cloned into a bacterial vector for DNA sequence determination. Ligations of the PCR fragments were carried out into the pCR2.1 T/A style cloning vector (Invitrogen) following the manufacturer's procedures using a 3: 1 insert to vector molar ratio. Two µl of the ligation reactions were used to transform the INVαF' competent cells (Invitrogen) as per the manufacturer's procedure. Plasmid clones containing DNA inserts were identified using diagnostic restriction enzyme digests, with EcoRI (New England Biolabs). The DNA sequence of plasmids containing the heavy chain inserts of the appropriate size (400 bp) was then determined. The final consensus DNA sequence of the heavy chain variable region of 11E10 is shown in Figure 6 with the CDRs indicated by underlining.

The light chain plasmid clones needed to be further characterized because the hybridoma cell line that expresses the "11E10" antibody was made by fusing mouse splenocytes with SP20 myeloma cells. The SP20 cell line transcribes a pseudogene for the kappa light chain. The pseudogene transcript, when converted to cDNA by RT-PCR, contains an AflIII restriction site. For this reason, the plasmid clones for the light chain variable region were digestes with AflIII and those products that did not cut were then submitted for DNA sequencing. The final consensus sequence of the light chain variable region is shown in Figure 6, with the CDRs indicated by underlining.

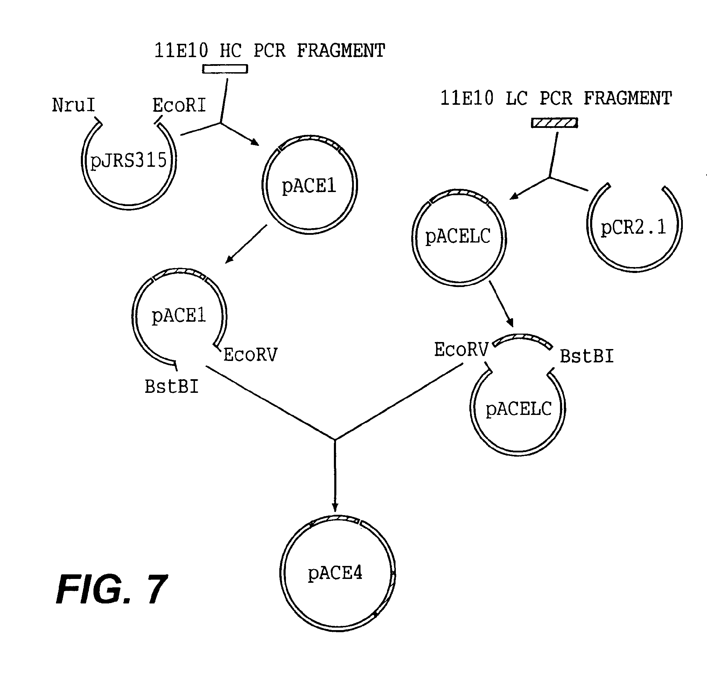

The variable region gene fragments were re-amplified by PCR using primers that adapted the fragments for cloning into the expression vector (see Figures 5 and 7). The heavy chain front primer (11E10HF, SEQ ID NO: 37) includes a 5'tail that encodes the C-terminus of the heavy chain leader and an NruI restriction site for cloning, while the heavy chain reverse primer (11E10HB, SEQ ID NO: 38) adds a 3'EcoRI restriction site for cloning. The light chain front primer (11E10LF, SEQ ID NO:39) includes a 5'tail that encodes the C-terminus of the light chain leader and an EcoRV restriction site at the N-terminus of the light chain variable region for cloning, while the light chain reverse primer (11E10LB, SEQ ID NO: 40) adds a 3'DNA sequence for the joining region-kappa exon splice junction followed by a BstBI restriction site for cloning. PCRs were performed as described above except, following a 5 min. incubation at 96°C, the PCR parameters were 30 thermal cycles of 96°C for 1 min., 62°C for 30 sec., and 70°C for 30 sec., followed by 5 min. at 72°C.

The heavy chain variable region PCR product was then subcloned into a mammalian expression plasmid vector (pJRS315, produced as set forth below in Example 5) for production of recombinant chimeric mouse/human antibody molecules. The resulting vector clone was designated pACE1. Before the light chain variable region could be subcloned into the mammalian expression vector, it was subcloned into the pCR2.1 T/A style cloning vector previously described. The resulting plasmid was designated pACELC and digeste, with BstBI/EcoRV, to cut out the light chain variable region. The variable region was then subcloned into the mammalian expression vector containing the "11E10" heavy chain variable region (pACE1). The final expression vector clone was designated pACE4. This vector results in the production of recombinant antibody molecules under the control of the CMV transcriptional promoters. The heavy chain molecules are direct cDNA constructs that fuse the variable region sequence directly into the human IgG 1 constant domain. The light chain molecules, on the other hand, have a mouse kappa intron region 3' of the variable region coding fragment. After splicing, the variable region becomes fused to a human kappa constant region exon (see Figure 7). The selectable marker for the vector in mammalian cells is neomycin (G418).

For the production of pACE1, the "1E10" heavy chain PCR product (approximately 400 bp) was digested with NruI and EcoRI (New England Biolabs), purified using a Qiaquick PCR Purification column (Qiagen), as described by the manufacturer, and ligated into NruI/EcoRI digested and gel-purified pJRS315, resulting in plasmid PACTE1 (see Figure 7). The "11E10" light chain PCR product (approximately 350 bp) was cloned into the T/A cloning vector as per manufacturer's instructions. The resulting clone, pACELC, was digested with EcoRV and BstBI (New England Biolabs) and the light chain fragment was gel-purified. This fragment was then ligated into the EcoRV/BstBI digested and gel-purified pACE1, resulting in plasmid pACE4 (see Figure 7). The sequence of the heavy and light chain variable regions was verified prior to mammalian cell transfection.

### EXAMPLE 5. Construction of the expression vector pJRS315.

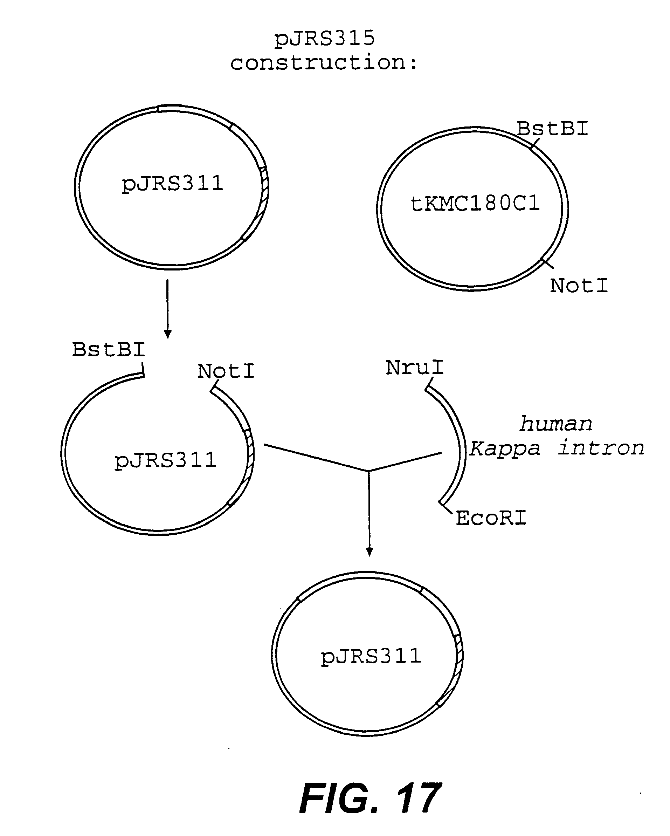

The plasmid pJRS315 is the expression plasmid into which the variable regions of the 11E10 antibody were cloned. This plasmid is a derivative of a basic expression vector, plasmid pSUN15, that contains no antibody variable region coding information, pJRS315 was created using DNA fragment ligations and site directed mutagenesis steps. The result was a vector that expresses both antibody chains with CMV promoter driven transcription (see Figure 17). Neomycin resistance serves as the dominant selectable marker for transfection of mammalian cells. In addition, it has been designed to allow convenient cloning of any light chain variable region as an EcoRV/BstBI fragment, any heavy chain variable region as a NruI/EcoRI fragment, and any heavy chain constant domain as an EcoRI/NotI fragment. These restriction sites were chosen because they occur rarely (if ever) in human and mouse variable regions. There is a mouse J region/kappa intron fragment fused to a human kappa exon so that after post-transcriptional splicing a mouse/human chimeric kappa light chain is produced.

The backbone of the vector was the plasmid pCDNA3 (Invitrogen). This plasmid was cut with HindIII/XhoI and a "light chain polylinker" DNA fragment was inserted to create the starting "light chain vector". This linker contained the restriction sites HindIII, KpnI, ClaI, PmlI, EcoRV, XmaI, BamHI and XhoI to facilitate subsequent cloning steps to create the plasmid pCDNA3.LCPL. A SmaI/BclI DNA fragment containing a light chain leader, anti-CKMB kappa light chain genomic fragment, and 3'UTR was cloned into the EcoRV/BamHI sites of pCDNA3.LCPL. The mouse kappa intron, exon and 3'UTR in this fragment was derived from LCPXK2 received from Dr. Richard Near (Near, R. I. et al., Mol. Immunol. 27: 901-909, (1990)). Mutagenesis was then performed to eliminate an NruI (209), MluI (229), and BstBI (2962) and to introduce an NheI (1229) and a BamHI (1214) site to create pcDNA3mut.LCPL.LCVK (see Figure 12).

A second "heavy chain vector" was constructed from the pcDNA3mut.LCPL.LCVK plasmid by replacing the light chain expression region (HindIII/XhoI) with a "heavy chain polylinker" consisting of restriction sites HpaI, BspEI, EcoRV, KpnI, and XhoI. This plasmid was digested with EcorRV and KpnI. A SmaI/KpnI digested DNA fragment containing a heavy chain leader and an anti-CKMB IgG2b mouse heavy chain genomic fragment was then ligated into the EcoRV/KpnI digested plasmid. A KpnI/SalI oligonucleotide fragment containing a 3'UTR and a NotI upstream of the SalI site was subsequently cloned into the KpnI/XhoI digested plasmid (knocking out the XhoI site), to create the plasmid pCDNA3mut.HCPL.HCV2b (see Figure 13).

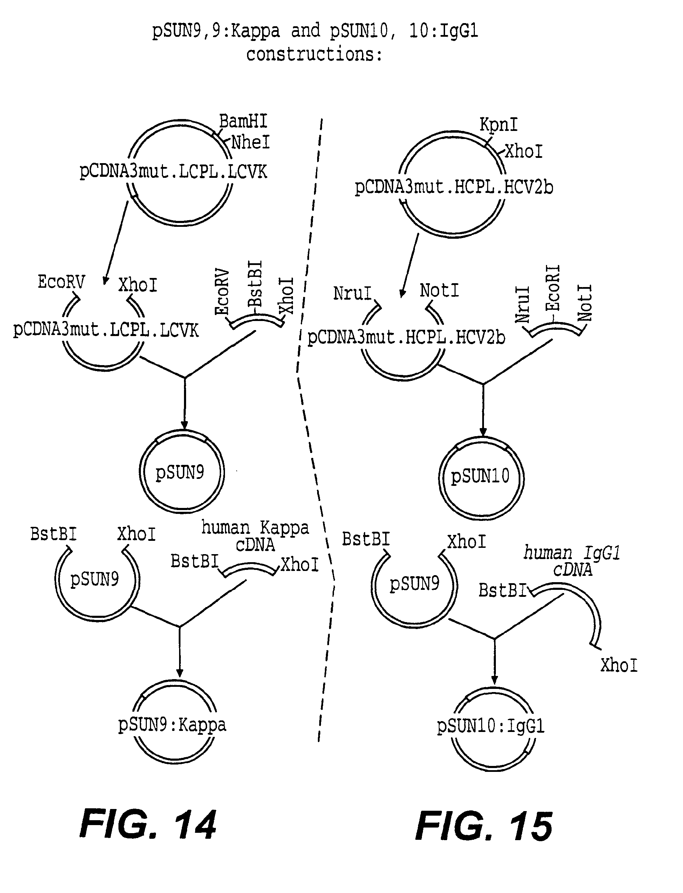

From this point, two vectors were created that did not have any of the anti-CKMB variable or constant domain DNA sequences. This was done by cutting the plasmid pcDNA3mut.LCPL.LCVK with EcoRV/XhoI and inserting a linker oligonucleotide fragment containing EcoRV, BstBI, and XhoI sites to create pSLJN9 (see Figure 14). In a similar way, the anti-CKMB fragment in pCDNA3mut.HCPL.HCV2b (NruI/NotI) was replaced by a linker oligonucleotide fragment containing NruI, EcoRI and NotI sites to create PSUN 10 (see Figure 15). A human kappa light chain constant domain was then cloned into pSUN9 as a BstBI/XhoI fragment, and a human IgG1 constant domain was cloned into pSUN10 as a EcoRI/NotI fragment.

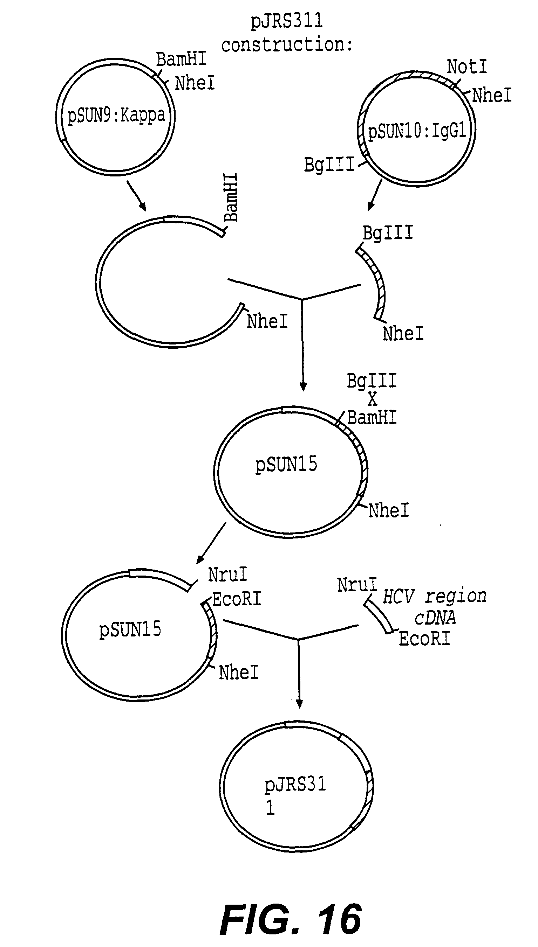

A BglII/NheI fragment from the human heavy chain vector was then cloned into the human light chain vector cut with BamHI/NheI to create pSUN15 (see Figure 16).

The plasmid pJRS315 was then constructed using pSUN15 through the following process. A heavy chain variable region from another, unrelated, hybridoma cell line (approximately 400 bp) was digested with NruI and EcoRI (New England Biolabs), purified using a Qiaquick PCR Purification column (Qiagen), as described by the manufacturer, and ligated into NruI/EcoRI digested and gel-purified pSUN15, resulting in plasmid pJRS311 (see Figure 16).

At this point, a BstBI/NotI (New England Biolabs) DNA fragment containing a mouse kappa J-kappa intron fragment fused to a human kappa exon fragment was digested and gel-purified from the vector tKMC180C2. This fragment was ligated into the backbone of pJRS311 digested with BstBI/NotI and gel-purified resulting in the plasmid pJRS315 (see Figure 17).

### EXAMPLE 6. Stable production of recombinant chimeric mouse/human "11E10" antibody.

### A. Transfection of NSO Cells

The plasmid pACE4 was transfected into NSO cells using electroporation. The plasmid was linearized with a PvuI restriction enzyme digestion. 40 µg of digested plasmid DNA was mixed with 7x10⁶ cells in a total volume of 400 µL and incubated at room temperature with gentle agitation for one minute. 10 µL of DMSO (Sigma) were added to a final concentration of 1.25%. The cells/DNA/DMSO mix was transferred to a cold 0.4 centimeter cuvette and subjected to one pulse of 250 volts, 960µF. The cells were transferred to one well of a six-well plate containing 5 ml of non-selective media supplemented with DMSO (final concentration of 1.25%). After 24 hours at 37°C and 10% CO₂ the cells were plated out into 96-well microtiter plates. As colonies appeared, the supernatants were assayed for the production of "humanized" antibody and for the capability for the expressed antibody to bind to the Stx2 toxin.

### B. Assay for Antibody Production

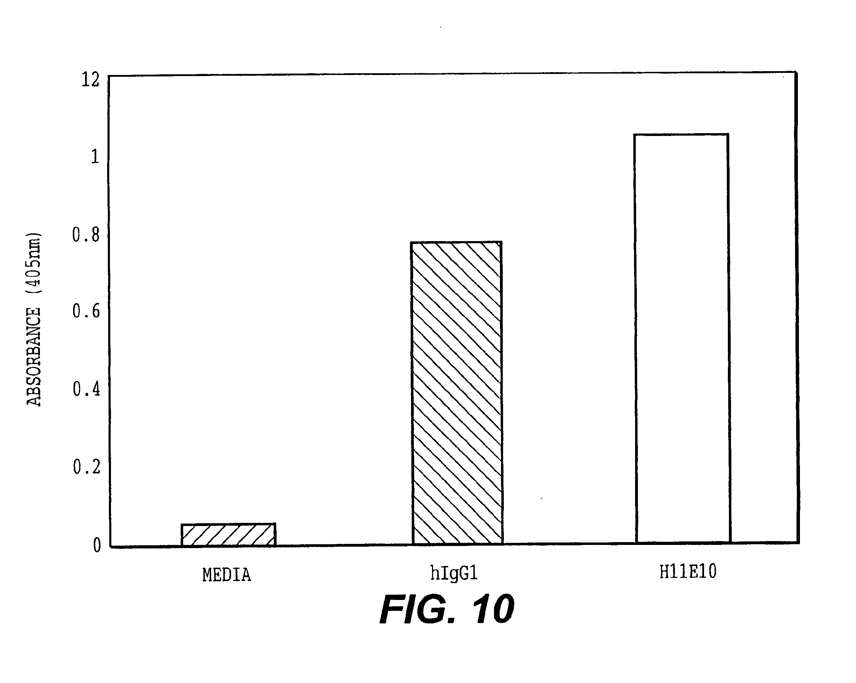

Antibody production and activity assays for the stable transfectants were performed in 8-well strips from 96-well microtiter plates (Maxisorp F8; Nunc, Inc.) coated with a 1: 500 dilution of goat anti-Human F(ab')₂ anti-IgG antibody (Southern Biotechnology) using a bicarbonate coating buffer, pH 8.5. The plates were covered with pressure sensitive film (Falcon, Becton Dickinson) and incubated overnight at 4°C. Plates are then washed once with wash solution (imidazole/NaCl/0.4% Tween-20). 100 µL of culture supernatant was then applied and allowed to incubate for 30 minutes on a plate rotator at room temperature. The plates were washed five times with wash solution (imidazole/NaCl/0.4% Tween-20). A goat anti-human kappa-HRP (Southern Biotechnology) conjugate was diluted 1: 800 in the sample/conjugate diluent and 100 µL was added to the samples, then incubated on a plate rotator for 30 minutes at room temperature. The samples were washed as above and then incubated with 100 µL per well of ABTS developing substrate (Kirkgaard & Perry Laboratories) and the absorbance value at 405 nm was determined using an automated microtiter plate ELISA Reader (Ceres UV900H1, BioTek Instruments, Winooski, Vermont). This assay (see Figure 10) demonstrates that the transfection of cells with this plasmid construct results in cells producing a molecule containing both human IgG and kappa domains.

The supernatants were then assayed for the ability of the expressed antibodies to bind to EHEC Stx2 toxin. The activity assay was performed, as above, using plates coated at 1 µg/ml with Stx2 toxin (obtained as in Example 7 from Dr. O'Brien's lab) in a bicarbonate coating buffer, pH 8.5. This assay demonstrates that the transfection of cells with this plasmid construct can result in the production of a humanized chimeric version of the 11E10 mouse monoclonal antibody which effectively binds Shiga toxin type 2 (Figure 11).

Together, these assays demonstrate that the transfection of cells with this plasmid construct can result in the production of a stable cell line that produces a humanized chimeric version of the 11E10 mouse hybridoma antibody.

EXAMPLE 7. Verifying Biological and Immunological Efficacy of Humanized Antibodies to Shiga toxin.

### A. Vero Cell Cytotoxicity Assay

The efficacy of the humanized antibodies to Shiga toxin obtained as detailed in Examples 1 through 6 was determined by assaying their ability to protect Vero cells from toxin. Cytotoxicity assays were performed essentially as described by Gentry and Dalrymple, J. Clin. Microbiol, 12: 361-366 (1980). Briefly, toxin was obtained from cultures of *E*. *coli* K-12 strains that contained either plasmid pLPSH3 (encodes Stx; J. Infect. Disease 164: 344-352 (1991)) or pMJ 100 (encodes Stx2; Inf. and Immunity, 57: 3743-3750 (1989)). Bacteria were disrupted by sonic lysis and clarified by centrifugation. The extracts were serially diluted in tissue culture medium (Dulbecco modified Eagle medium containing 10% fetal calf serum, 0.8 mM glutamine, 500 U of penicillin G per ml, and 500 mg of streptomycin per ml). One hundred microliters of 10-fold dilutions of the lysates were added to microtiter plate wells containing about 10⁴ Vero cells in 100 µl of medium. The tissue culture cells were incubated at 37°C in 5% CO₂ for 48 hours and then fixed and stained with crystal violet. The intensity of color of the fixed and stained cells was measured with a Titertek reader at 620 nm. Incubation without antibody provides a standard toxicity curve for the Stx.

### B. Antisera Neutralization Assay

Humanized mouse antibodies obtained according to the methods described in Examples 1 through 6 were tested for toxin neutralization. Neutralization of cytotoxic activity was described in great detail in Schmitt et al., Infect. and Immun., 59: 1065-1073 (1991). Briefly, lysates were incubated with serial dilutions of the humanized mouse antibodies at 37°C for 2 hours. One hundred microliters of the samples were then added to Vero cells as described above.

**TABLE 1: Vero Cell Neutralization Assay**

| Antibody | Required ng to Neut. 1 CD₅₀* |
|---|---|
| Mouse 13C4 | 25 |
| Humanized 13C4 | 26 |
| Mouse 11E10 | 2.7 |
| Humanized 11E10 | 82.8 |

| | |
|---|---|
| **(1CD₅₀ = 1pg pure toxin)* | |

### EXAMPLE 8. Passive Immunization with Humanized Antibodies

### A. Protection against injection with crude Stx1 toxin.

At day zero, groups of five CD-1 mice were injected intraperitoneally (0.1 ml) with antibody H13C4 (humanized α-Stx1B), 13C4 (mouse α-Stx1B), H11E10 (humanized α-Stx2A), or phosphate buffered saline (PBS). These injections were repeated at day 1. The mice were then challenge by intravenous injection with crude Stx1 toxin (obtained as described in Example 7) at doses of 1.7 x 10⁵ or 1.7 x 10⁶ CD₅₀. These doses of toxin were chosen following preliminary experiments with varying amounts of toxin. Mice were monitored for 21 days.

The results (Table 2) show clearly that the injected antibodies protect the mice against at least 10 times the normal lethal toxin dose. As a negative control, the antibodies to Stx2 did not protect against Stx1 toxin challenge, indicating that the protection was specific to the toxin/antibody pair, and was not an artifact of the antibody preparation process.

**TABLE 2: CD-1 Mice Injected Intravenously with Crude Stx1**

| Antibody (dose/mouse) | #LD₅₀ Protected Against | µg/kg of Ab to Protect from 1LD₅₀ |
|---|---|---|
| Murine 13C4 (1.4 µg) | 10 | 6.1 |
| Humanized 13C4 (4.1µg) | 20 | 8.9 |
| Humanized 11E10 (232 µg) | 0 | No protection |

| | | |
|---|---|---|
| * *(1 LD₅₀ = 30 ng crude Stx1)* | | |

### B. Protection against oral infection with Stx2-producing EHEC trains.

Two different strains of mice and bacteria were used in these studies to test efficacy against both Stx2 and Stx2-variant. DBA/2J mice were challenge with EHEC strain 86-24 (0157: H7, Stx2⁺) and CD-1 mice were challenge with strain B2F1 (091: H21, Stx2-variant⁺). While *E. coli* strain B2F1 is normally fatal to both mice trains, *E.coli* strain 86-24 is fatal to DBA/2J mice, while CD-1 mice will survive 86-24 infection.

At day zero, antibody H11E10 (humanized α-Stx2A) or 11E10 (mouse α -Stx2A) was injecte intraperitoneally (0.1 ml) into groups of four or five mice. Control groups included mice that had received antibody 13C4 (mouse α -Stx1B), mice that had received 11E10 ascites fluid (mouse α -Stx2A), or mice that had received PBS instead of antibody. Mice were given streptomycin (5g/L) in their drinking water to decrease normal intestinal flora and their food was removed. Streptomycin resistant derivatives of strains 86-24 (0157: H7, Stx2⁺) and B2F1 (091:H21, Stx2-variant⁺) were grown overnight in L broth.

The following day (day 1) the mice received a second injection of test antibody, control antibody, or PBS. The mice were immediately fed 10¹⁰ CFU of 86-24 that had been pelleted and resuspended in 20% sucrose or 10³ CFU of B2F1 that had been serially diluted in 20% sucrose. Food was returned to the cage and the mice were monitored for 21 days.

### (CFU=Colony Forming Units)

As shown in Table 3, immunization of the mice with the either the murine or the humanized anti-Stx2 antibodies resulted in complete protection from a lethal oral dose of EHEC. Immunization with mouse 13C4 antibody, prepared in the same way but immunoreactive with the Stx I toxin instead of the Stx2 toxin did not protect the mice from challenge with 86-24, a result that indicates the immunospecificity of the response.

**TABLE 3: DBA/2J Mice Fed 101° CFU 0157 (Stx2)**

| Antibody (dose/mouse) | Survivors |
|---|---|
| Murine 13C4 (1.4µg) | 0/5 |
| Murine 11E10 (1.0 µg) | 5/5 |
| Humanized 11E10 (1.0µg) | 5/5 |

Similar results are illustrated in Table 4, where the more resistant CD-1 mice were fed the B2F1 *E. coli* strain which produces Stx2-variant toxin. Without treatment with antibody, mortality was total, but both the mouse and the humanized antibodies protected against Stx2-variant in a dose dependent manner.

**TABLE 4: CD-1 Mice Fed 103 CFU 091: A21 (Stx2-variant)**

| Antibody (dose/mouse) | Survivors |
|---|---|
| PBS | 0/5 |
| Murine 11E10 (8.7 µg) | 1/4* |
| Murine 11E10 (6. 45 mg) | 5/5 |
| Humanized 11E10 (23.2 µg) | 0/5 |
| Humanized 11E10 (232 µg) | 5/5 |

| | |
|---|---|
| *Protective dose between 10.1 and 1.0 mg*/*kg* **Delayed mean time to death by 3.4 days* | |

### EXAMPLE 9. Treatment of disease caused by bacteria producing Shiga toxin.

Applicants further describe a variety of methods of treating, ameliorating, or preventing, the diseases and effects associated with exposure to Shiga toxin. Positive clinical responses in humans have been obtained with monoclonal antibodies, and one skilled in the art would know how to employ anti-Stx humanized monoclonal antibodies in humans. (See Fagerberg et al., Cancer Research, 55: 1824-27 (1995); Eur. J. Cancer, 2: 261-267 (1995)). The precise dosage of humanized anti-Shiga toxin antibody administered to a patient for treatment of these diseases will vary in accordance with factors appreciated by the typical clinician. These factors include (but are not limited to) size, age, overall health, the extent of infection, and other medications being administered to said patient. The development of a precise treatment regimen will require optimization through routine medical procedures well known to those in the medical arts. Examples of potential patient groups would include (but not be limited to) young children with bloody diarrhea but no white cells in the stool, patients with indications of HUS, patients with positive stool sample tests for Shiga toxin, siblings or daycare cohorts in contact with a case (as a passive preventative measure), and any patient with diarrhea (not necessarily bloody) that has been in contact with an identified case. A typical dosage of about 5mg/kg body weight of humanized 13C4 combine with about 10 mg/kg body weight of humanized 11E10 would be contemplated. This combined formulation could be administered to the patient twice to ensure effectiveness. Inclusion of both types of humanized antibodies together provides assurance that the patient will be protected against all types of Shiga toxin disclosed herein.

### SEQUENCE LISTING

<110> HENRY JACKSON FOUNDATION FOR THE ADVANCEMENT OF MILITARY MEDICINE
<120> HUMANIZED MONOCLONAL ANTIBODIES THAT PROTECT AGAINST SHIGA TOXIN INDUCED DISEASE
<130> 04995.0032-00304
<140>
   <141>
<160> 44
<170> PatentIn Ver. 2.0
<210> 1
   <211> 45
   <212> DNA
   <213> Artificial Sequence
<220>
   <223> Description of Artificial Sequence: Synthetic Oligonucleotide
<400> 1
   att tca ggc cca gcc ggc cat ggc cga rgt rma gct ksa kga gwc 45
<210> 2
   <211> 45
   <212> DNA
   <213> Artificial Sequence
<220>
   <223> Description of Artificial Sequence: Synthetic Oligonucleotide
<400> 2
   atttcaggcc cagccggcca tggccgargt ycarctkcar caryc 45
<210> 3
   <211> 45
   <212> DNA
   <213> Artificial Sequence
<220>
   <223> Description of Artificial Sequence: Synthetic Oligonucleotide
<400> 3
   atttcaggcc cagccggcca tggcccaggt gaagctksts gartc 45
<210> 4
   <211> 45
   <212> DNA
   <213> Artificial Sequence
<220>
   <223> Description of Artificial Sequence: Synthetic Oligonucleotide
<400> 4
   atttcaggcc cagccggcca tggccgargt rmagctksak gagwc 45
<210> 5
   <211> 45
   <212> DNA
   <213> Artificial Sequence
<220>
   <223> Description of Artificial Sequence: Synthetic Oligonucleotide
<400> 5
   atttcaggcc cagccggcca tggcccaggt bcarctkmar sartc 45
<210> 6
   <211> 20
   <212> DNA
   <213> Artificial Sequence
<220>
<223> Description of Artificial Sequence: Synthetic Oligonucleotide
<400> 6
   gaartavccc ttgaccaggc 20
<210> 7
   <211> 35
   <212> DNA
   <213> Artificial Sequence
<220>
   <223> Description of Artificial Sequence: Synthetic Oligonucleotide
<400> 7
   ggaggcggcg gttctgacat tgtgmtgwcm cartc 35
<210> 8
   <211> 35
   <212> DNA
   <213> Artificial Sequence
<220>
   <223> Description of Artificial Sequence: Synthetic Oligonucleotide
<400> 8
   ggaggcggcg gttctgatrt tkygatgacb carrc 35
<210> 9
   <211> 35
   <212> DNA
   <213> Artificial Sequence
<220>
   <223> Description of Artificial Sequence: Synthetic Oligonucleotide
<400> 9
   ggaggcggcg gttctgayat ymagatgacm cagwc 35
<210> 10
   <211> 35
   <212> DNA
   <213> Artificial Sequence
<220>
   <223> Description of Artificial Sequence: Synthetic Oligonucleotide
<400> 10
   ggaggcggcg gttctsaaat tgwkctsacy cagtc 35
<210> 11
   <211> 40
   <212> DNA
   <213> Artificial Sequence
<220>
   <223> Description of Artificial Sequence: Synthetic Oligonucleotide
<400> 11
   ttcataggcg gccgcactag tagcmcgttt cagytccarc 40
<210> 12
   <211> 40
   <212> DNA
   <213> Artificial Sequence
<220>
   <223> Description of Artificial Sequence: Synthetic Oligonucleotide
<400> 12
   ttcataggcg gccgcactag tagcmcgttt katytccarc 40
<210> 13
   <211> 23
   <212> DNA
   <213> Artificial Sequence
<220>
   <223> Description of Artificial Sequence: Synthetic Oligonucleotide
<400> 13
   gcacctccag atgttaactg ctc 23
<210> 14
   <211> 49
   <212> DNA
   <213> Artificial Sequence
<220>
   <223> Description of Artificial Sequence: Synthetic Oligonucleotide
<400> 14
   cttgatcgcg acagctacag gtgtccactc ccaggtgcag ctgcaggag 49
<210> 15
   <211> 36
   <212> DNA
   <213> Artificial Sequence
<220>
   <223> Description of Artificial Sequence: Synthetic Oligonucleotide
<400> 15
   ggtatggaat tctgaggaga ctgtgagagt ggtgcc 36
<210> 16
   <211> 36
   <212> DNA
   <213> Artificial Sequence
<220>
   <223> Description of Artificial Sequence: Synthetic Oligonucleotide
<400> 16
   ggttctgata tcgtgatgtc ccagtctcac aaattc 36
<210> 17
   <211> 42
   <212> DNA
   <213> Artificial Sequence
<220>
   <223> Description of Artificial Sequence: Synthetic Oligonucleotide
<400> 17
   gacatattcg aaaagtgtac ttacgtttca gctccagact gg 42
<210> 18
   <211> 366
   <212> DNA
   <213> Shigella dysenteriae
<400> 18 <210> 19
   <211> 122
   <212> PRT
   <213> Shigella dysenteriae
<400> 19
<210> 20
   <211> 324
   <212> DNA
   <213> Shigella dysenteriae
<400> 20
   gatatcgtga tgtcccagtc tcacaaattc atgtccacat cagtcggaga cagggtcagc 60
   atcacctgta aggccagcca ggatgtgggt actgctgttg cctggtatca gcagaatcca 120
   ggacaatctc ctaaatttct gatttactgg gcatccacac ggcacactgg agtccctgat 180
   cgcttcacag gcagtggatc tgggacagat ttcactctca ccattaccaa tgtgcagtct 240
   gaagacttgg cagattattt ctgtcagcaa tatagcagtt atcctctcac gttcggtgct 300
   gggaccagtc tggagctgaa acgt                                        324
<210> 21
   <211> 108
   <212> PRT
   <213> Shigella dysenteriae
<400> 21
   Asp Ile Val Met Ser Gln Ser His Lys Phe Met Ser Thr Ser Val Gly
   1               5                   10                  15
   Asp Arg Val Ser Ile Thr Cys Lys Ala Ser Gln Asp Val Gly Thr Ala
               20                  25                  30
   Val Ala Trp Tyr Gln Gln Asn Pro Gly Gln Ser Pro Lys Phe Leu Ile
           35                  40                  45
   Tyr Trp Ala Ser Thr Arg His Thr Gly Val Pro Asp Arg Phe Thr Gly
       50                  55                  60
   Ser Gly Ser Gly Thr Asp Phe Thr Leu Thr Ile Thr Asn Val Gln Ser
   65                  70                  75                  80
   Glu Asp Leu Ala Asp Tyr Phe Cys Gln Gln Tyr Ser Ser Tyr Pro Leu
                   85                  90                  95
   Thr Phe Gly Ala Gly Thr Ser Leu Glu Leu Lys Arg
               100                 105
<210> 22
   <211> 45
<212> DNA
   <213> Artificial Sequence
<220>
   <223> Description of Artificial Sequence: Synthetic Oligonucleotide
<400> 22
   atttcaggcc cagccggcca tggccgargt rmagctksak gagwc 45
<210> 23
   <211> 45
   <212> DNA
   <213> Artificial Sequence
<220>
   <223> Description of Artificial Sequence: Synthetic Oligonucleotide
<400> 23
   atttcaggcc cagccggcca tggccgargt ycarctkcar caryc 45
<210> 24
   <211> 45
   <212> DNA
   <213> Artificial Sequence
<220>
   <223> Description of Artificial Sequence: Synthetic Oligonucleotide
<400> 24
   atttcaggcc cagccggcca tggcccaggt gaagctksts gartc 45
<210> 25
   <211> 45
   <212> DNA
   <213> Artificial Sequence
<220>
   <223> Description of Artificial Sequence: Synthetic Oligonucleotide
<400> 25
   atttcaggcc cagccggcca tggccgavgt gmwgctkgtg gagwc 45
<210> 26
   <211> 45
   <212> DNA
   <213> Artificial Sequence
<220>
   <223> Description of Artificial Sequence: Synthetic Oligonucleotide
<400> 26
   atttcaggcc cagccggcca tggcccaggt bcarctkmar sartc 45
<210> 27
   <211> 20
   <212> DNA
   <213> Artificial Sequence
<220>
   <223> Description of Artificial Sequence: Synthetic Oligonucleotide
<400> 27
   gaartavccc ttgaccaggc 20
<210> 28
   <211> 35
   <212> DNA
   <213> Artificial Sequence
<220>
   <223> Description of Artificial Sequence: Synthetic Oligonucleotide
<400> 28
   gctgccaccg ccacctgmrg agacdgtgas tgarg 35
<210> 29
   <211> 35
   <212> DNA
   <213> Artificial Sequence
<220>
   <223> Description of Artificial Sequence: Synthetic Oligonucleotide
<400> 29
   gctgccaccg ccacctgmrg agacdgtgas mgtrg 35
<210> 30
   <211> 35
   <212> DNA
   <213> Artificial Sequence
<220>
   <223> Description of Artificial Sequence: Synthetic Oligonucleotide
<400> 30
   gctgccaccg ccacctgmrg agacdgtgas cagrg 35
<210> 31
   <211> 35
   <212> DNA
   <213> Artificial Sequence
<220>
   <223> Description of Artificial Sequence: Synthetic Oligonucleotide
<400> 31
   ggaggcggcg gttctgacat tgtgmtgwcm cartc 35
<210> 32
   <211> 35
   <212> DNA
   <213> Artificial Sequence
<220>
   <223> Description of Artificial Sequence: Synthetic Oligonucleotide
<400> 32
   ggaggcggcg gttctgatrt tkygatgacb carrc 35
<210> 33
   <211> 35
   <212> DNA
   <213> Artificial Sequence
<220>
   <223> Description of Artificial Sequence: Synthetic Oligonucleotide
<400> 33
   ggaggcggcg gttctgayat ymagatgacm cagwc 35
<210> 34
   <211> 35
   <212> DNA
   <213> Artificial Sequence
<220>
   <223> Description of Artificial Sequence: Synthetic Oligonucleotide
<400> 34
   ggaggcggcg gttctsaaat tgwkctsacy cagtc 35
<210> 35
   <211> 40
   <212> DNA
   <213> Artificial Sequence
<220>
   <223> Description of Artificial Sequence: Synthetic Oligonucleotide
<400> 35
   ttcataggcg gccgcactag tagcmcgttt cagytccarc 40
<210> 36
   <211> 40
   <212> DNA
   <213> Artificial Sequence
<220>
   <223> Description of Artificial Sequence: Synthetic Oligonucleotide
<400> 36
   ttcataggcg gccgcactag tagcmcgttt katytccarc 40
<210> 37
   <211> 52
   <212> DNA
   <213> Artificial Sequence
<220>
   <223> Description of Artificial Sequence: Synthetic Oligonucleotide
<400> 37
   atatactcgc gacagctaca ggtgtccact ccgaagtcca actgcaacag cc 52
<210> 38
   <211> 34
   <212> DNA
   <213> Artificial Sequence
<220>
   <223> Description of Artificial Sequence: Synthetic Oligonucleotide
<400> 38
   attaatgaat tctgcggaga cggtgagagt ggtc 34
<210> 39
   <211> 29
   <212> DNA
   <213> Artificial Sequence
<220>
   <223> Description of Artificial Sequence: Synthetic Oligonucleotide
<400> 39
   ttaaatgata tcgtgctgtc acaatctcc 29
<210> 40
   <211> 45
   <212> DNA
   <213> Artificial Sequence
<220>
   <223> Description of Artificial Sequence: Synthetic Oligonucleotide
<400> 40
   taatcgttcg aaaagtgtac ttacgtttca gttccagctt ggtcc 45
<210> 41
   <211> 339
   <212> DNA
   <213> Shigella dysenteriae
<400> 41
   gacattgtgc tgtcacaatc tccatcctcc ctagttgtgt cagttggaga gaaggttact 60
   atgagctgca agtctagtca gagcctttta tatagtagaa atcaaaagaa ctacttggcc 120
   tggtaccagc agaaaccagg gcagtctcct aaagtgctga tttactgggc atctactagg 180
   gaatctgggg tccctgatcg cctcacaggc agtggatctg ggacagattt cactctcacc 240
   atcagcagtg tgaaggctga agacctggca gtttattact gtcagcaata ttatagttat 300
   ccgctcacgt tcggtgctgg gaccaagctg gagctgaaa 339
<210> 42
   <211> 113
   <212> PRT
   <213> Shigella dysenteriae
<400> 42
   Asp Ile Val Leu Ser Gln Ser Pro Ser Ser Leu Val Val Ser Val Gly
   1               5                   10                  15
   Glu Lys Val Thr Met Ser Cys Lys Ser Ser Gln Ser Leu Leu Tyr Ser
               20                  25                  30
   Arg Asn Gln Lys Asn Tyr Leu Ala Trp Tyr Gln Gln Lys Pro Gly Gln
           35                  40                  45
   Ser Pro Lys Val Leu Ile Tyr Trp Ala Ser Thr Arg Glu Ser Gly Val
       50                  55                  60
   Pro Asp Arg Leu Thr Gly Ser Gly Ser Gly Thr Asp Phe Thr Leu Thr
   65                  70                  75                  80
   Ile Ser Ser Val Lys Ala Glu Asp Leu Ala Val Tyr Tyr Cys Gln Gln
                   85                  90                  95
   Tyr Tyr Ser Tyr Pro Leu Thr Phe Gly Ala Gly Thr Lys Leu Glu Leu
               100                 105                 110
   Lys
<210> 43
   <211> 357
   <212> DNA
   <213> Shigella dysenteriae
<400> 43
   gaagtccaac tgcaacagcc tggacctgag ctggagaagc ctggcgcttc agtgaaacta 60
   tcctgcaagg cttctggtta ctctttcact gactacaaca tgaactgggt gaagcagaac 120
   aatggagaga gccttgagtg gattggaaaa attgatcctt actatggtgg tcctagctac 180
   aaccagaagt tcaaggacaa ggccacattg actgtagaca agtcttccag cacagcctac 240
   atgcagttca agagcctgac atctgaggac tctgcagtct attactgtac aagaggggga 300
   aatagggact ggtacttcga tgtgtggggc gcagggacca cgctcaccgt ctccgca    357
<210> 44
   <211> 119
   <212> PRT
   <213> Shigella dysenteriae
<400> 44
   Glu Val Gln Leu Gln Gln Pro Gly Pro Glu Leu Glu Lys Pro Gly Ala
   1               5                   10                  15
   Ser Val Lys Leu Ser Cys Lys Ala Ser Gly Tyr Ser Phe Thr Asp Tyr
               20                  25                  30
   Asn Met Asn Trp Val Lys Gln Asn Asn Gly Glu Ser Leu Glu Trp Ile
           35                  40                  45
   Gly Lys Ile Asp Pro Tyr Tyr Gly Gly Pro Ser Tyr Asn Gln Lys Phe
       50                  55                  60
   Lys Asp Lys Ala Thr Leu Thr Val Asp Lys Ser Ser Ser Thr Ala Tyr
   65                  70                  75                  80
   Met Gln Phe Lys Ser Leu Thr Ser Glu Asp Ser Ala Val Tyr Tyr Cys
                   85                  90                  95
   Thr Arg Gly Gly Asn Arg Asp Trp Tyr Phe Asp Val Trp Gly Ala Gly
               100                 105                 110
   Thr Thr Leu Thr Val Ser Ala
           115

## Claims

1. A pharmaceutical composition comprising a first humanized monoclonal antibody that binds to Shiga toxin type 1 (Stx1) protein and a second humanized monoclonal antibody that binds to Shiga toxin type 2 (Stx2) protein, wherein the first antibody comprises a human immunoglobulin constant region and comprises the immunoglobulin heavy chain and light chain variable regions as set forth in SEQ ID NOs: 19 and 21 and wherein the second antibody comprises a human immunoglobulin constant region and comprises the immunoglobulin heavy chain and light chain variable regions as set forth in SEQ ID NOs: 42 and 44.

2. A pharmaceutical composition comprising a first humanized monoclonal antibody that binds to Stx1 and a second humanized monoclonal antibody that binds to Stx2 antigen, wherein the variable region of the second antibody comprises the variable region of the murine 11E10 antibody with ATCC Accession No. CRL 1907 and the variable region of the first antibody comprises the variable region of the murine 13C4 antibody with ATCC Accession No. CKL 1794.

3. The pharmaceutical composition of claim 1 or 2, wherein the first or second antibody comprises a human constant region selected from the group consisting of IgG, IgA, and IgM.

4. The pharmaceutical composition of claim 3, wherein the human constant region of the first or second monoclonal antibody is IgG.

5. The pharmaceutical composition of claim 3, wherein the human constant region of the first or second monoclonal antibody is IgGI-kappa.

6. The pharmaceutical composition of any one of claims 1 to 5, further comprising a pharmaceutically acceptable carrier or diluent.

7. The use of a composition comprising a first humanized monoclonal antibody that binds to Stx1 protein and a second humanized monoclonal antibody that binds to Stx2 protein, wherein the first antibody comprises a human immunoglobulin constant region and comprises the immunoglobulin heavy chain and light chain variable regions as set forth in SEQ ID NOs: 19 and 21 and wherein the second antibody comprises a human immunoglobulin constant region and comprises the immunoglobulin heavy chain and light chain variable regions as set forth in SEQ ID NOs: 42 and 44 in the manufacture of a medicament for the treatment or prevention of an infection resulting in hemolytic uremia syndrome caused by enterohemorrhagic *Escherichia coli* or other Shiga toxin producing bacteria.

8. The use of a composition comprising a first humanized monoclonal antibody that binds to Stx1 and a second humanized monoclonal antibody that binds to Stx2 antigen, wherein the variable region of the second antibody comprises the variable region of the murine 11E10 antibody with ATCC Accession No. CRL 1907and the variable region of the first antibody comprises the variable region of the murine 13C4 antibody with ATCC Accession No. CKL 1794 in the manufacture of a medicament for the treatment or prevention of an infection resulting in hemolytic uremia syndrome caused by enterohemorrhagic *Escherichia coli* or other Shiga toxin producing bacteria.

9. The use according to claim 7 or 8, wherein the first or second antibody comprises a human constant region selected from the group consisting of IgG, IgA, and IgM.

10. The use according to claim 9, wherein the human constant region of the first or second monoclonal antibody is IgG, preferably IgGI-kappa.

11. A composition comprising a first humanized monoclonal antibody that binds to Stx1 protein and a second humanized monoclonal antibody that binds to Stx2 protein, wherein the first antibody comprises a human immunoglobulin constant region and comprises the immunoglobulin heavy chain and light chain variable regions as set forth in SEQ ID NOs: 19 and 21 and wherein the second antibody comprises a human immunoglobulin constant region and comprises the immunoglobulin heavy chain and light chain variable regions as set forth in SEQ ID NOs: 42 and 44 for treating or preventing an infection resulting in hemolytic uremia syndrome caused by enterohemorrhagic *Escherichia coli* or other Shiga toxin producing bacteria.

12. A composition comprising a first humanized monoclonal antibody that binds to Stx1 and a second humanized monoclonal antibody that binds to Stx2 antigen, wherein the variable region of the second antibody comprises the variable region of the murine 11E10 antibody with ATCC Accession No. CRL 1907 and the variable region of the first antibody comprises the variable region of the murine 13C4 antibody with ATCC Accession No. CKL 1794, for treating or preventing an infection resulting in hemolytic uremia syndrome caused by enterohemorrhagic *Escherichia coli* or other Shiga toxin producing bacteria.

13. The composition to claim 11 or 12, wherein the first or second antibody comprises a human constant region selected from the group consisting of IgG, IgA, and IgM.

14. The composition according to claim 13, wherein the human constant region of the first or second monoclonal antibody is IgG, preferably IgGI-kappa.

## Patentansprüche

1. Pharmazeutische Zusammensetzung, umfassend einen ersten humanisierten monoklonalen Antikörper, der an Shiga-Toxin-Typ-I-(Stx1)-Protein bindet, und einen zweiten humanisierten monoklonalen Antikörper, der an Shiga-Toxin-Typ-2-(Stx2)-Protein bindet, wobei der erste Antikörper eine konstante Region eines Human-Immunglobulins umfasst und die Schwerkette des Immunglobulins und variable Regionen der Leichtkette wie in SEQ ID Nummern 19 und 21 dargelegt umfasst, und wobei der zweite Antikörper eine konstante Region des Human-Immunglobulins umfasst und die Schwerkette und variable Regionen der Leichtkette des Immunglobulins wie in SEQ ID Nummern 42 und 44 dargelegt umfasst.

2. Pharmazeutische Zusammensetzung, umfassend einen ersten humanisierten monoklonalen Antikörper, der an Stx1 bindet, und einen zweiten humanisierten monoklonalen Antikörper, der an Stx2-Antigen bindet, wobei die variable Region des zweiten Antikörpers die variable Region des 11E10-Maus-Antikörpers mit ATCC-Akzessionsnummer CRL 1907 umfasst und die variable Region des ersten Antikörpers die variable Region des 13C4-Maus-Antikörpers mit ATCC-Akzessionsnummer CKL 1794 umfasst.

3. Pharmazeutische Zusammensetzung gemäß Anspruch 1 oder 2, wobei der erste oder zweite Antikörper eine menschliche konstante Region ausgewählt aus der Gruppe bestehend aus IgG, IgA und IgM umfasst.

4. Pharmazeutische Zusammensetzung gemäß Anspruch 3, wobei die menschliche konstante Region des ersten oder zweiten monoklonalen Antikörpers IgG ist.

5. Pharmazeutische Zusammensetzung gemäß Anspruch 3, wobei die menschliche konstante Region des ersten oder zweiten monoklonalen Antikörpers IgGI-kappa ist.

6. Pharmazeutische Zusammensetzung nach einem der Ansprüche 1 bis 5, weiterhin umfassend einen pharmazeutisch unbedenklichen Träger oder ein Verdünnungsmittel.

7. Verwendung einer Zusammensetzung, umfassend einen ersten humanisierten monoklonalen Antikörper, der an Stx1-Protein bindet, und einen zweiten humanisierten monoklonalen Antikörper, der an Stx2-Protein bindet, wobei der erste Antikörper eine konstante Region eines menschlichen Immunglobulins umfasst und die Schwerkette und variable Regionen der Leichtkette des Immunglobulins wie in SEQ ID-Nummern 19 und 21 dargelegt umfasst, und wobei der zweite Antikörper eine konstante Region eines menschlichen Immunglobulins umfasst und die Schwerkette und variable Regionen der Leichtkette des Immunglobulins wie in SEQ ID-Nummern 42 und 42 dargelegt umfasst, bei der Herstellung eines Arzneimittels zur Behandlung oder Vorbeugung einer Infektion, die zu hämolytischem urämischem Syndrom, hervorgerufen durch enterohämorrhagische *Escherichia coli* oder andere Shiga-Toxin produzierende Bakterien, führt.

8. Verwendung einer Zusammensetzung, umfassend einen ersten humanisierten monoklonalen Antikörper, der an Stx1 bindet, und einen zweiten humanisierten monoklonalen Antikörper, der an Stx2-Antigen bindet, wobei die variable Region des zweiten Antikörpers die variable Region des 11E10-Maus-Antikörpers mit ATCC-Akzessionsnummer CRL 1907 umfasst, und die variable Region des ersten Antikörpers die variable Region des 13C4-Maus-Antikörpers mit ATCC-Akzessionsnummer CKL 1794 umfasst, bei der Herstellung eines Arzneimittels zur Behandlung oder Vorbeugung einer Infektion, die zu hämolytischem urämischem Syndrom, hervorgerufen durch enterohämorrhagische *Escherichia coli* oder andere Shiga-Toxin produzierende Bakterien, führt.

9. Verwendung gemäß Anspruch 7 oder 8, wobei der erste oder zweite Antikörper eine menschliche konstante Region ausgewählt aus der Gruppe bestehend aus IgG, IgA und IgM umfasst.

10. Verwendung gemäß Anspruch 9, wobei die menschliche konstante Region des ersten oder zweiten monoklonalen Antikörpers IgG, vorzugsweise IgGI-kappa ist.

11. Zusammensetzung, umfassend einen ersten humanisierten monoklonalen Antikörper, der an Stx1-Protein bindet, und einen zweiten humanisierten monoklonalen Antikörper, der an Stx2-Protein bindet, wobei der erste Antikörper eine konstante Region eines Human-Immunglobulins umfasst und die Schwerkette und variable Regionen der Leichtkette des Immunglobulins wie in SEQ ID Nummern 19 und 21 dargelegt umfasst, und wobei der zweite Antikörper eine konstante Region des Human-Immunglobulins umfasst und die Schwerkette und variable Regionen der Leichtkette des Immunglobulins wie in SEQ ID Nummern 42 und 44 dargelegt umfasst, zur Behandlung oder Vorbeugung einer Infektion, die zu hämolytischem urämischem Syndrom, hervorgerufen durch enterohämorrhagische *Escherichia coli* oder andere Shiga-Toxin produzierende Bakterien, führt.

12. Zusammensetzung, umfassend einen ersten humanisierten monoklonalen Antikörper, der an Stx1 bindet, und einen zweiten humanisierten monoklonalen Antikörper, der an Stx2-Antigen bindet, wobei die variable Region des zweiten Antikörpers die variable Region des 11E10-Maus-Antikörpers mit ATCC-Akzessionsnummer CRL 1907 umfasst und die variable Region des ersten Antikörpers die variable Region des 13C4-Maus-Antikörpers mit ATCC-Akzessionsnummer CKL 1794 umfasst, zur Behandlung oder Vorbeugung einer Infektion, die zu hämolytischem urämischem Syndrom, hervorgerufen durch enterohämorrhagische *Escherichia coli* oder andere Shiga-Toxin produzierende Bakterien, führt.

13. Zusammensetzung gemäß Anspruch 11 oder 12, wobei der erste oder zweite Antikörper eine menschliche konstante Region ausgewählt aus der Gruppe bestehend aus IgG, IgA und IgM umfasst.

14. Pharmazeutische Zusammensetzung gemäß Anspruch 13, wobei die menschliche konstante Region des ersten oder zweiten monoklonalen Antikörpers IgG, bevorzugt IgGI-kappa ist.

## Revendications

1. Composition pharmaceutique comprenant un premier anticorps monoclonal humanisé qui se lie à la protéine qu'est la toxine Shiga de type 1 (Stx1) et un second anticorps monoclonal humanisé qui se lie à la protéine qu'est la toxine Shiga de type 2 (Stx2), le premier anticorps comportant une région constante d'immunoglobuline humaine et les régions variables de chaîne lourde et de chaîne légère d'immunoglobuline décrites par les séquences N° 19 et 21 et le second anticorps comportant une région constante d'immunoglobuline humaine et les régions variables de chaîne lourde et de chaîne légère d'immunoglobuline décrites par les séquences N° 42 et 44.

2. Composition pharmaceutique comprenant un premier anticorps monoclonal humanisé qui se lie à l'antigène Stx1 et un second anticorps monoclonal humanisé qui se lie à l'antigène Stx2, la région variable du second anticorps étant constituée de la région variable de l'anticorps 11E10 murin dont le numéro d'accès ATCC est CRL 1907 et la région variable du premier anticorps étant constituée de la région variable de l'anticorps 13C4 murin dont le numéro d'accès ATCC est CKL 1794.

3. Composition pharmaceutique conforme à la revendication 1 ou 2, dans laquelle le premier ou le second anticorps comporte une région constante humaine choisie dans l'ensemble constitué par les IgG, IgA et IgM.

4. Composition pharmaceutique conforme à la revendication 3, dans laquelle la région constante humaine du premier ou du second anticorps monoclonal est une région constante d'IgG.

5. Composition pharmaceutique conforme à la revendication 3, dans laquelle la région constante humaine du premier ou du second anticorps monoclonal est une région constante d'IgG1-kappa.

6. Composition pharmaceutique conforme à l'une des revendications 1 à 5 et comprenant en outre un véhicule ou un diluant pharmacologiquement admissible.

7. Utilisation d'une composition qui comprend un premier anticorps monoclonal humanisé qui se lie à la protéine Stx1 et un second anticorps monoclonal humanisé qui se lie à la protéine Stx2, le premier anticorps comportant une région constante d'immunoglobuline humaine et les régions variables de chaîne lourde et de chaîne légère d'immunoglobuline décrites par les séquences N° 19 et 21 et le second anticorps comportant une région constante d'immunoglobuline humaine et les régions variables de chaîne lourde et de chaîne légère d'immunoglobuline décrites par les séquences N° 42 et 44, dans la fabrication d'un médicament destiné au traitement ou à la prévention d'une infection provoquant le syndrome hémolytique et urémique dû à une souche entérohémorragique d'*Escherichia coli* ou à une autre bactérie productrice de toxine Shiga.

8. Utilisation d'une composition comprenant un premier anticorps monoclonal humanisé qui se lie à l'antigène Stx1 et un second anticorps monoclonal humanisé qui se lie à l'antigène Stx2, la région variable du second anticorps étant constituée de la région variable de l'anticorps 11E10 murin dont le numéro d'accès ATCC est CRL 1907 et la région variable du premier anticorps étant constituée de la région variable de l'anticorps 13C4 murin dont le numéro d'accès ATCC est CKL 1794, dans la fabrication d'un médicament destiné au traitement ou à la prévention d'une infection se traduisant par le syndrome hémolytique et urémique dû à une souche entérohémorragique d'*Escherichia coli* ou à une autre bactérie productrice de toxine Shiga.

9. Utilisation conforme à la revendication 7 ou 8, dans laquelle le premier ou le second anticorps comporte une région constante humaine choisie parmi les régions constantes des IgG, IgA et IgM.

10. Utilisation conforme à la revendication 9, dans laquelle la région constante humaine du premier ou du second anticorps monoclonal est une région constante d'IgG, de préférence d'IgG1-kappa.

11. Composition qui comprend un premier anticorps monoclonal humanisé qui se lie à la protéine Stx1 et un second anticorps monoclonal humanisé qui se lie à la protéine Stx2, le premier anticorps comportant une région constante d'immunoglobuline humaine et les régions variables de chaîne lourde et de chaîne légère d'immunoglobuline décrites par les séquences N° 19 et 21 et le second anticorps comportant une région constante d'immunoglobuline humaine et les régions variables de chaîne lourde et de chaîne légère d'immunoglobuline décrites par les séquences N° 42 et 44, destinée au traitement ou à la prévention d'une infection provoquant le syndrome hémolytique et urémique dû à une souche entérohémorragique d'*Escherichia coli* ou à une autre bactérie productrice de toxine Shiga.

12. Composition comprenant un premier anticorps monoclonal humanisé qui se lie à l'antigène Stx1 et un second anticorps monoclonal humanisé qui se lie à l'antigène Stx2, la région variable du second anticorps étant constituée de la région variable de l'anticorps 11E10 murin dont le numéro d'accès ATCC est CRL 1907 et la région variable du premier anticorps étant constituée de la région variable de l'anticorps 13C4 murin dont le numéro d'accès ATCC est CKL 1794, destinée au traitement ou à la prévention d'une infection se traduisant par le syndrome hémolytique et urémique dû à une souche entérohémorragique d'Escherichia coli ou à une autre bactérie productrice de toxine Shiga.

13. Composition conforme à la revendication 11 ou 12, dans laquelle le premier ou le second anticorps comporte une région constante humaine choisie parmi les régions constantes des IgG, IgA et IgM.

14. Composition conforme à la revendication 13, dans laquelle la région constante humaine du premier ou du second anticorps monoclonal est une région constante d'IgG, de préférence d'IgG1-kappa.
